# EUROPEAN PATENT SPECIFICATION

(11) **EP 3 976 809 B1**
(45) Date of publication and mention of the grant of the patent: **05.06.2024**
(21) Application number: 20727647.8
(22) Date of filing: 26.05.2020
(51) Int. Cl.: C12P 19/34, C12N 9/12

(54) **VARIANTS OF TERMINAL DEOXYNUCLEOTIDYL TRANSFERASE AND USES THEREOF**
VARIANTEN VON TERMINALER DESOXYNUKLEOTIDYLTRANSFERASE UND VERWENDUNGEN DAVON
VARIANTS DE DÉSOXYNUCLÉOTIDYLE TRANSFÉRASE TERMINALE ET LEURS UTILISATIONS

(30) Priority: 28.05.2019 EP 19177018; 28.05.2019 US 201916423972
(43) Date of publication of application: 06.04.2022
(73) Proprietor: DNA Script, 94270 Le Kremlin-Bicêtre (FR); INSTITUT PASTEUR, 75724 Paris Cedex 15 (FR); Centre national de la recherche scientifique, 75016 Paris (FR)
(72) Inventor: CHAMPION, Elise, 75013 Paris (FR); SOSKINE, Mikhael, 95130 Franconville (FR); YBERT, Thomas, 75012 Paris (FR); DELARUE, Marc, 78000 Versailles (FR)
(74) Representative: Plasseraud IP
(86) International application number: PCT/EP2020/064524
(87) International publication number: WO 2020/239737

(56) References cited:
- WO-A1-2018/102818
- WO-A2-2017/216472
- BAOLI YANGS ET AL: "Mutational Analysis of Residues in the Nucleotide Binding Domain of Human Terminal Deoxynucleotidyl Transferase*", THE JOURNAL OF BIOLOGICAL CHEMISTRY, vol. 269, 22 April 1994 (1994-04-22), pages 11859-11868, XP055570430,

## Description

### FIELD OF THE INVENTION

The invention relates to variants of Terminal deoxynucleotidyl Transferase (TdT) and uses thereof for the enzymatic synthesis of nucleic acid sequences without template. More particularly, the present invention relates to such variants suitable to incorporate modified nucleotides, for the synthesis of nucleic acid molecules with determined or controlled sequences.

### BACKGROUND

Methods for *de novo* chemical synthesis of nucleic acids based on solid-phase phosphoramidite chemistry have been largely used and refined over the past 40 years. The technique consists of a four-step chain elongation cycle that adds one base per cycle onto a growing oligonucleotide chain attached to a solid support matrix. Although it has been the method of choice to synthesize nucleic acids during the past decades, this technology has some notable limitations: It requires the use of multiple solvents and reagents, and due to limitations in chemical reaction efficiency, the length of synthetic oligonucleotides typically do not exceed 150-200 bases. Moreover, these short fragments need to be further assembled to provide the desired DNA sequence.

One alternative to chemical synthesis consists in using template independent DNA polymerases that will add reversible terminator modified nucleotides to a growing single stranded chain of nucleic acids. This allows the addition of one type of nucleotide per cycle in a controlled fashion.

Some native enzymes are able to act on natural nucleotides in the absence of template and so can catalyze the synthesis of nucleic acids in an uncontrolled fashion. However, they are particularly inefficient to incorporate modified nucleotides and more particularly reversible terminator modified nucleotides. Efforts have been made to develop new DNA polymerases able to act on modified nucleotides but the resulting enzymes are not fully satisfactory in terms of performances for the synthesis of any type of nucleic acids.

So far, only few DNA polymerases that can act efficiently on single strand DNA (without the use of template) have been identified. The most characterized polymerase having such template-independent activity is the Terminal deoxynucleotidyl Transferase (TdT). TdT enzymes have been extensively used to modify single stranded DNA for various types of applications including biotechnology, biomedical research and synthetic biology. However, native TdT is poorly able to use modified nucleotides.

Several attempts to develop modified TdT with acceptable performance for the incorporation of modified nucleotides have been carried over. However, the performances of the incorporation of such modified nucleotides is still a limiting factor. Incorporation efficiency is the key parameter driving the overall purity and yield of synthesis. These two characteristics of the synthesis process have a significant impact of quality, turnaround time and cost of nucleic acid products.

There is therefore a need to develop improved TdT capable to use modified nucleotides in the absence of template, for developing efficient and cost-effective methods for the nucleic acid synthesis.

### SUMMARY OF THE INVENTION

By working on TdT for *de novo* synthesis of polynucleotides with controlled sequence and without the use of a template, the inventors have discovered that some targeted amino acid residues of the catalytic domain of the TdT may be specifically modified to improve the ability of such modified TdT for synthesizing polynucleotides. More particularly, the inventors have developed modified TdTs with targeted amino acid substitution(s) that lead to improve the enzymatic synthesis of polynucleotides and to reduce the overall cost of synthesizing polynucleotides. The inventors previously developed variants of murine TdT (SEQ ID NO: 1 or SEQ ID NO:2). By further working on TdT, the inventors have now developed new TdT variants.

In some embodiments, each of the modified TdTs presents one or more targeted amino acids substitution as compared to wild-type TdTs (such as SEQ ID NOs 10, 12, 14, 16, 18, 20, 22, 24, 26, 28, 30, 32 or 34) and N-terminal truncated versions thereof that comprise a TdT catalytic domain (such as SEQ ID NOs 11, 13, 15, 17, 19, 21, 23, 25, 27, 29, 31, 33 or 35). In some embodiments, each of the modified TdTs of the invention possesses an amino acid sequence having a specified percent sequence identity with a catalytic domain of aTdT (such as SEQ ID NOs 11, 13, 15, 17, 19, 21, 23, 25, 27, 29, 31, 33 or 35) and having one or more specified amino acid substitution(s). The template-independent polymerases of the invention allow the enzymatic synthesis of polynucleotides at a faster rate, with less expense and higher quality.

It is therefore an object of the invention to provide variants of Terminal deoxynucleotidyl Transferase (TdT) which comprise an amino acid sequence of a TdT catalytic domain or of a percent sequence identity of a TdT catalytic domain, such as set forth in SEQ ID NOs 11, 13, 15, 17, 19, 21, 23, 25, 27, 29, 31, 33 or 35, with at least an amino acid substitution at position corresponding to residue C173 (with respect to the amino acid numbering of SEQ ID NO: 11), or functionally equivalent residue, is capable of synthesizing a nucleic acid fragment without template and is capable of incorporating a modified nucleotide, such as 3'-O- modified nucleotide, into a nucleic acid fragment. Particularly, the modified nucleotide is incorporated onto a free 3'-hydroxyl of the nucleic fragment.

More particularly, it is an object of the present invention to provide terminal deoxynucleotidyl transferase (TdT) variants comprising an amino acid sequence at least 90% identical to SEQ ID NO: 11, 13, 15, 17, 19, 21, 23, 25, 27, 29, 31, 33 or 35 with a substitution at position corresponding to residue C173 with respect to SEQ ID NOs 11, 13, 17, 19, 21, 29 or 31, or at position corresponding to residue C172 with respect to SEQ ID NO: 15, or at position corresponding to residue C178 with respect to SEQ ID NO: 23, or at position corresponding to residue C174 with respect to SEQ ID NO: 25, or at position corresponding to residue C171 with respect to SEQ ID NO: 27, or at position corresponding to residue C182 with respect to SEQ ID NO: 33, or at position corresponding to residue C176 with respect to SEQ ID NO: 35, and wherein the amino acid sequence comprises a further substitution of an arginine residue at position 207 with respect to SEQ ID NOs 11, 13, 17, 19, 23, 29 of 31, or of a functionally equivalent arginine residue selected from an arginine at position 206 with respect to SEQ ID NO: 15, or a arginine at position 208 with respect to SEQ ID NOs 21 or 25, or arginine at position 205 with respect to SEQ ID NO: 27, or arginine at position 216 with respect to SEQ ID NO: 33, or a arginine at position 210 with respect to SEQ ID NO: 35, wherein the TdT variant (i) is capable of synthesizing a nucleic acid fragment without a template and (ii) is capable of incorporating a modified nucleotide into a nucleic acid fragment.

In some embodiments, the above percent identity value is at least 95% identity with the indicated SEQ ID NOs; in some embodiments, the above percent identity value is at least 97% identity; in some embodiments, the above percent identity value is at least 98% identity; in some embodiments, the above percent identity value is at least 99% identity.

Advantagesously, in regard to (ii), such modified nucleotide is a 3'-O-modified nucleotide that may be incorporated onto a free 3'-hydroxyl of the nucleic acid fragment. The 3'-O-modified nucleotide may comprise a 3'-O-NH2-nucleoside triphosphate, a 3'-O-azidomethyl-nucleoside triphosphate, a 3'-O-allyl-nucleoside triphosphate, a 3'O-(2-nitrobenzyl)-nucleoside triphosphate, or a 3'-O-propargyl-nucleoside triphosphate.

Part of the invention are variants only which have at least the positions 173 with respect to SEQ ID NOs 11, 13, 17, 19, 21, 29 or 31 and 207 with respect to SEQ ID NOs 11, 13, 17, 19, 23, 29 of 31 and functionally equivalent positions substituted. Any other disclosure is a disclosure which is not part of the invention. The substitution is selected from

C313G/R/P/A/V/S/N/Q/D with respect to SEQ ID NO: 10; or C173G/R/P/A/V/S/N/Q/D with respect to SEQ ID NO: 11; or C302G/R/P/A/V/S/N/Q/D with respect to SEQ ID NO: 12; or C173G/R/P/A/V/S/N/Q/D with respect to SEQ ID NO: 13; or C302G/R/P/A/V/S/N/Q/D with respect to SEQ ID NO: 14; or C172G/R/P/A/V/S/N/Q/D with respect to SEQ ID NO: 15; or C304G/R/P/A/V/S/N/Q/D with respect to SEQ ID NO: 16; or C173G/R/P/A/V/S/N/Q/D with respect to SEQ ID NO: 17; or C304G/R/P/A/V/S/N/Q/D with respect to SEQ ID NO: 18; or C173G/R/P/A/V/S/N/Q/D with respect to SEQ ID NO: 19; or C293G/R/P/A/V/S/N/Q/D with respect to SEQ ID NO: 20; or C174G/R/P/A/V/S/N/Q/D with respect to SEQ ID NO: 21; or C282G/R/P/A/V/S/N/Q/D with respect to SEQ ID NO: 22; or C173G/R/P/A/V/S/N/Q/D with respect to SEQ ID NO: 23; or C304G/R/P/A/V/S/N/Q/D with respect to SEQ ID NO: 24; or C174G/R/P/A/V/S/N/Q/D with respect to SEQ ID NO: 25; or C300G/R/P/A/V/S/N/Q/D with respect to SEQ ID NO: 26; or C171G/R/P/A/V/S/N/Q/D with respect to SEQ ID NO: 27; or C305G/R/P/A/V/S/N/Q/D with respect to SEQ ID NO: 28; or C173G/R/P/A/V/S/N/Q/D with respect to SEQ ID NO: 29; or C302G/R/P/A/V/S/N/Q/D with respect to SEQ ID NO: 30; or C173G/R/P/A/V/S/N/Q/D with respect to SEQ ID NO: 31; or C313G/R/P/A/V/S/N/Q/D with respect to SEQ ID NO: 32; or C182G/R/P/A/V/S/N/Q/D with respect to SEQ ID NO: 33; or C271G/R/P/A/V/S/N/Q/D with respect to SEQ ID NO: 34; or C176G/R/P/A/V/S/N/Q/D with respect to SEQ ID NO: 35.

In a further embodiment, the substitution is selected from
C302G/R with respect to SEQ ID NO: 4; or C302G/R with respect to SEQ ID NO: 9; or C313G/R with respect to SEQ ID NO: 10; or C173G/R with respect to SEQ ID NO: 11; or C302G/R with respect to SEQ ID NO: 12; or C173G/R with respect to SEQ ID NO: 13; or C302G/R with respect to SEQ ID NO: 14; or C172G/R with respect to SEQ ID NO: 15; or C304G/R with respect to SEQ ID NO: 16; or C173G/R with respect to SEQ ID NO: 17; or C304G/R with respect to SEQ ID NO: 18; or C173G/R with respect to SEQ ID NO: 19; or C293G/R with respect to SEQ ID NO: 20; or C173G/R with respect to SEQ ID NO: 21; or C282G/R with respect to SEQ ID NO: 22; or C173G/R with respect to SEQ ID NO: 23; or C304G/R with respect to SEQ ID NO: 24; or C174G/R with respect to SEQ ID NO: 25; or C300G/R with respect to SEQ ID NO: 26; or C171G/R with respect to SEQ ID NO: 27; or C305G/R with respect to SEQ ID NO: 28; or C173G/R with respect to SEQ ID NO: 29; or C302G/R with respect to SEQ ID NO: 30; or C173G/R with respect to SEQ ID NO: 31; or C313G/R with respect to SEQ ID NO: 32; or C182G/R with respect to SEQ ID NO: 33; or C271G/R with respect to SEQ ID NO: 34; or C176G/R with respect to SEQ ID NO: 35.

The disclosure is directed to compositions comprising TdT variants comprising amino acid sequence having at least 90 percent identity, or in some embodiments, at least 95 percent identity, at least 97 percent identity, or in some embodiments, at least 98 percent identity, or at least 99% identity with a reference or wild type TdT sequence selected from the group consisting of SEQ ID NOs: 11, 13, 15, 17, 19, 21, 23, 25, 27, 29, 31, 33 and 35, wherein such TdT variants have a mutation selected from C173G/R/P/A/V/S/N/Q/D, preferably C173G/R (wherein the amino acid residue number is with respect to SEQ ID NO: 11, or an equivalent residue number of SEQ ID NOs 13, 15, 17, 19, 21, 23, 25, 27, 29, 31, 33 or 35) (i) is capable of synthesizing a nucleic acid fragment without a template and (ii) such TdT variants incorporate 3'-O-modified nucleoside triphosphates with greater efficiency, or at a higher rate, than the reference or wild type TdT.

The disclosure provides truncated variants of Terminal deoxynucleotidyl Transferase (TdT) each of which comprises an amino acid sequence with at least 90 percent identity to any of SEQ ID NOs 11, 13, 15, 17, 19, 21, 23, 25, 27, 29, 31, 33 or 35 with at least two amino acid substitutions, preferably at least three amino acid substitutions, selected from M63R/Q, L131P, C173G/R, R207L/N, D250V, R325P/N and E328N/L/T/S, (wherein residue numbers are with respect to SEQ ID NO: 11or with respect to their functionally equivalent residues numbers in SEQ ID NOs 13, 15, 17, 19, 21, 23, 25, 27, 29, 31, 33 or 35), (i) is able to synthesize a nucleic acid fragment without a template and (ii) is able to incorporate a modified nucleotide into the nucleic acid fragment, for example, a 3'-O-reversibly blocked deoxynucleoside triphosphate onto a free 3'-hydroxyl of a nucleic acid fragment.

The above percent sequence identity value is at least 95, 96, 97, 98 or 99 percent identity with the specified sequences.

It is another object of the invention to provide a nucleic acid molecule encoding a variant of a TdT as defined above and/or an expression vector comprising such nucleic acid molecule, and/or a host cell comprising such nucleic acid molecule or expression vector.

It is a further object of the invention to provide a process for producing a variant of TdT according to the invention, wherein a host cell as defined above is cultivated under culture conditions allowing the expression of the nucleic acid encoding said variant, and wherein the variant is optionally retrieved.

The invention further relates to the use of a variant of TdT, for synthesizing a nucleic acid molecule without template, by the successive addition of one or more 3'O-modified nucleotides to a nucleic acid fragment. In some embodiments, such methods comprise the steps of (a) providing an initiator comprising an oligonucleotide having a free 3'-hydroxyl; (b) reacting under enzymatic extension conditions a TdT variant of the invention with the initiator or an extended initiator in the presence of a 3'-O-reversibly blocked nucleoside. In some embodiments, such method further includes steps of (c) deblocking the extended initiators to form extended initiators with free 3'-hydroxyls and (d) repeating steps (b) and (c) until a nucleic acid molecule of a predetermined sequence is synthesized.

It is also an object of the invention to provide a process for synthesizing a nucleic acid molecule without template, comprising a step of contacting a nucleic acid primer with both at least one nucleotide, preferably at least one 3'O-modified nucleotide, and a variant of TdT according to the invention.

The present invention further provides a kit for performing a nucleotide incorporation reaction comprising a variant of TdT according to the invention, and one or more nucleotides, preferably one or more 3'O-modified nucleotides, and optionally at least one nucleic acid primer.

### DESCRIPTION OF THE INVENTION

The DNA polymerase families are divided into seven families based on their sequence homology and crystal structure. Among them, the polymerases of PolX family represent a wide variety of polymerases from replicative polymerases to terminal transferase enzymes. Polymerases from PolX family are present across a very wide range of eukaryotic organisms. Polymerases from the PolX family are implicated in a vast variety of biological processes and in particular in DNA damage repair mechanisms or error correction mechanisms. The PolX family regroups polymerase β (Pol β), µ (Pol µ), λ (Pol λ), IV from yeast (Pol IV) and the Terminal deoxynucleotidyl Transferase (TdT). TdT is naturally implicated in DNA repair and maintenance mechanisms. In particular, TdT has the unique ability to conserve a nucleotide polymerization activity even in absence of template strand. In specific conditions and with natural nucleotides, TdT is able to elongate DNA fragments with several hundred nucleotides, in absence of any complementary strand. However, wild type TdT is totally unable to efficiently incorporate sugar-modified nucleotides.

It is thus the purpose of the present invention to provide variants of TdT with targeted mutation(s) that allow them to incorporate modified nucleotides into a nucleic fragment during synthesize of said nucleotide fragment. More particularly, the inventors have identified specific amino acid residues that may be advantageously substituted, alone or in combination, to improve the ability of the enzyme to synthesize nucleic acid fragments of various length and with pre-determined sequence, including by using modified nucleotides. The invention is defined by the claims only.

### Definitions

As used therein, the terms *"mutant"* and *"variant"* may be used interchangeably to refer to polypeptides related to or derived from SEQ ID NO: 11, 12, 13, 14, 15, 16, 17, 18, 19, 20, 21, 22, 23, 24, 25, 26 27, 28, 29, 30, 31, 32, 33, 34 or 35 and comprising a modification or an alteration, i.e., a substitution, insertion, and/or deletion, at one or more (e.g., several) positions and having both a polymerase activity without template and ability to incorporate 3'-O-modified nucleoside triphosphates into a nucleic acid chain . The variants may be obtained by various techniques well known in the art. In particular, examples of techniques for altering the DNA sequence encoding the wild-type protein, include, but are not limited to, site-directed mutagenesis, random mutagenesis and synthetic oligonucleotide construction. Mutagenesis activities consist in deleting, inserting or substituting one or several amino-acids in the sequence of a protein or in the case of the invention of a polymerase. Targeted amino-acids could be concomitant or distributed along the whole sequence of the polymerase. Specific motifs or structural features could be targeted for example.

The terms *"modification"* or *"alteration"* as used herein in relation to a position or amino acid mean that the amino acid in the specific position has been modified compared to the amino acid of the wild-type protein.

A *"substitution"* means that an amino acid residue is replaced by another amino acid residue. Preferably, the term *"substitution"* refers to the replacement of an amino acid residue by another selected from the naturally-occurring standard 20 amino acid residues, rare naturally occurring amino acid residues (e.g. hydroxyproline, hydroxylysine, allohydroxylysine, 6-N-methylysine, N-ethylglycine, N-methylglycine, N-ethylasparagine, allo-isoleucine, N-methylisoleucine, N-methylvaline, pyroglutamine, aminobutyric acid, ornithine, norleucine, norvaline), and non-naturally occurring amino acid residue, often made synthetically, (e.g. cyclohexyl-alanine). Preferably, the term *"substitution"* refers to the replacement of an amino acid residue by another selected from the naturally-occurring standard 20 amino acid residues. The sign "+" indicates a combination of substitutions.

The amino acids are herein represented by their one-letter or three-letters code according to the following nomenclature: A: alanine (Ala); C: cysteine (Cys); D: aspartic acid (Asp); E: glutamic acid (Glu); F: phenylalanine (Phe); G: glycine (Gly); H: histidine (His); I: isoleucine (Ile); K: lysine (Lys); L: leucine (Leu); M: methionine (Met); N: asparagine (Asn); P: proline (Pro); Q: glutamine (Gln); R: arginine (Arg); S: serine (Ser); T: threonine (Thr); V: valine (Val); W: tryptophan (Trp ) and Y: tyrosine (Tyr).

In the present document, the following terminology is used to designate a substitution: L238A denotes that amino acid residue (Leucine, L) at position 238 of the parent sequence is changed to an Alanine (A). A132V/I/M denotes that amino acid residue (Alanine, A) at position 132 of the parent sequence is substituted by one of the following amino acids: Valine (V), Isoleucine (I), or Methionine (M). The substitution can be a conservative or non-conservative substitution. Examples of conservative substitutions are within the groups of basic amino acids (arginine, lysine and histidine), acidic amino acids (glutamic acid and aspartic acid), polar amino acids (glutamine, asparagine and threonine), hydrophobic amino acids (methionine, leucine, isoleucine, cysteine and valine), aromatic amino acids (phenylalanine, tryptophan and tyrosine), and small amino acids (glycine, alanine and serine).

As used herein, the terms *"sequence identity"* or *"identity"* refer to the number (or fraction expressed as a percentage %) of matches (identical amino acid residues) between two polypeptide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithm (e.g. Needleman and Wunsch algorithm; Needleman and Wunsch, 1970) which aligns the sequences optimally over the entire length, while sequences of substantially different lengths are preferably aligned using a local alignment algorithm (e.g. Smith and Waterman algorithm (Smith and Waterman, 1981) or Altschul algorithm (Altschul et al., 1997; Altschul et al., 2005)). Alignment for purposes of determining percent amino acid sequence identity can be achieved in various ways that are within the skill in the art, for instance, using publicly available computer software available on internet web sites such as http://blast.ncbi.nlm.nih.gov/ or http://www.ebi.ac.uk/Tools/emboss/. Those skilled in the art can determine appropriate parameters for measuring alignment, including any algorithm needed to achieve maximal alignment over the full length of the sequences being compared. For purposes herein, % amino acid sequence identity values refer to values generated using the pair wise sequence alignment program EMBOSS Needle, that creates an optimal global alignment of two sequences using the Needleman-Wunsch algorithm, wherein all search parameters are set to default values, i.e. Scoring matrix = BLOSUM62, Gap open = 10, Gap extend = 0.5, End gap penalty = false, End gap open = 10 and End gap extend = 0.5.

Herein, the terms *"peptide", "polypeptide", "protein", "enzyme*", refer to a chain of amino acids linked by peptide bonds, regardless of the number of amino acids forming said chain.

Unless otherwise specified, the positions disclosed in the present application are numbered by reference to the amino acid sequence set forth in a specified SEQ ID NO.

### Variants of TdT

The present invention provides variants of TdT enzyme that can be used for synthesizing polynucleotides of predetermined sequences, such as DNA or RNA, without the use of template strand. The TdT variants of the invention allow modified nucleotides, and more particularly 3'O-modified nucleotides, to be used in an enzyme-mediated method of polynucleotide synthesis, such as described by Hiatt et al, U.S. patent 5763594.

In some embodiments of the invention, "modified Terminal desoxyribonucleotidyl Transferase", "modified TdT", "variants of Terminal desoxyribonucleotidyl Transferase" and "variants of TdT" refer to enzymes shares at least 90% identity with SEQ ID NOS: 2, 11, 13, 15, 17, 19, 21, 23, 25, 27, 29, 31, 33, 35, excepting at least amino acid residue substitutions at postion 173 and 207. In still other embodiments, the variant of TdT comprises an amino acid sequence that shares at least 95% identity with SEQ ID NOS: 2, 11, 13, 15, 17, 19, 21, 23, 25, 27, 29, 31, 33, 35, excepting at least the amino acid substitutions at positions 173 and 207. In still other embodiments, the variant of TdT comprises an amino acid sequence that shares at least 97%, 98% or 99% identity with SEQ ID NOS: 2, 11, 13, 15, 17, 19, 21, 23, 25, 27, 29, 31, 33, 35, excepting at least the amino acid substitutions at positions 173 and 207.

In some cases, variants of the present invention may be described according to their mutations on specific residues, whose positions are determined by alignment with or reference to a SEQ ID NO.

By "functionally equivalent residue" is meant a residue in a given sequence of a TdT having an identical functional role as compared to a corresponding residue in a functionally equivalent sequence.In the context of the invention, each sequence of TdT selected from SEQ ID NO: 11, 13, 15, 17, 19, 21, 23, 25, 27, 29, 31, 33 or 35 may be considered as a "functionally equivalent sequence" of any one of the other sequences.

In some embodiments, the invention comprises a variant of Terminal deoxynucleotidyl Transferase (TdT) that comprises an amino acid sequence having at least 90% preferably at

least 95%, 97%, 98% or 99% identity with an amino acid sequence selected from SEQ ID NO: 11, 13, 15, 17, 19, 21, 23, 25, 27, 29, 31, 33 or 35, with at least an amino acid substitution at position corresponding to a functionally equivalent residue of residue C173 with respect to SEQ ID NO:11, (i) is able to synthesize a nucleic acid fragment without template and (ii) is able to incorporate a 3'-O-modified nucleoside triphosphate, such as a 3'-O-blocked nucleoside triphosphate, into the nucleic fragment.

Indeed, the inventors have discovered that such substitution has a great impact on both surface and interaction properties of the enzyme with nucleotides, which may allow incorporation of 3'O-modified nucleotides in a nucleic acid sequence.

Further embodiments of TdT variants of the invention are listed as entries in Tables 1A through 1C (single substitutions), Tables 2A through 2C (two substitutions), Tables 3A through 3C (three substitutions), and Tables 4A through 4F (four substitutions), wherein each such variant TdT is defined by the indicated SEQ ID NO in the righthand column modified by the substitution(s) listed in the lefthand column of the same row as the SEQ ID NO. A "non-wild type" substitution means that the substitution may be any amino acid except for the amino acid at the indicated position in the wild type sequence, or equivalently, the sequence of the indicated SEQ ID NO.

**Table1A: TdT variants at position C173 (SEQ ID NO: 11) or functionally equivalent positions of the indicated SEQ ID NO**

| Non-wild type substitution at | SEQ ID NO |
|---|---|
| C313 | 10 |
| C173 | 11 |
| C302 | 12 |
| C173 | 13 |
| C302 | 14 |
| C172 | 15 |
| C304 | 16 |
| C173 | 17 |
| C304 | 18 |
| C173 | 19 |
| C293 | 20 |
| C173 | 21 |
| C282 | 22 |
| C178 | 23 |
| C304 | 24 |
| C 174 | 25 |
| C300 | 26 |
| C171 | 27 |
| C305 | 28 |
| C173 | 29 |
| C302 | 30 |
| C173 | 31 |
| C313 | 32 |
| C182 | 33 |
| C271 | 34 |
| C176 | 35 |

**Table1B: Further TdT variants at position C173 (SEQ ID NO: 11) or functionally equivalent positions of the indicated SEQ ID NO**

| Substitution | SEQ ID NO |
|---|---|
| C313/G/R/P/A/V/S/N/Q/D | 10 |
| C173/G/R/P/A/V/S/N/Q/D | 11 |
| C302/G/R/P/A/V/S/N/Q/D | 12 |
| C173/G/R/P/A/V/S/N/Q/D | 13 |
| C302/G/R/P/A/V/S/N/Q/D | 14 |
| C172/G/R/P/A/V/S/N/Q/D | 15 |
| C304/G/R/P/A/V/S/N/Q/D | 16 |
| C173/G/R/P/A/V/S/N/Q/D | 17 |
| C304/G/R/P/A/V/S/N/Q/D | 18 |
| C173/G/R/P/A/V/S/N/Q/D | 19 |
| C293/G/R/P/A/V/S/N/Q/D | 20 |
| C173/G/R/P/A/V/S/N/Q/D | 21 |
| C282/G/R/P/A/V/S/N/Q/D | 22 |
| C178/G/R/P/A/V/S/N/Q/D | 23 |
| C304/G/R/P/A/V/S/N/Q/D | 24 |
| C174/G/R/P/A/V/S/N/Q/D | 25 |
| C300/G/R/P/A/V/S/N/Q/D | 26 |
| C171/G/R/P/A/V/S/N/Q/D | 27 |
| C305/G/R/P/A/V/S/N/Q/D | 28 |
| C173/G/R/P/A/V/S/N/Q/D | 29 |
| C302/G/R/P/A/V/S/N/Q/D | 30 |
| C173/G/R/P/A/V/S/N/Q/D | 31 |
| C313/G/R/P/A/V/S/N/Q/D | 32 |
| C182/G/R/P/A/V/S/N/Q/D | 33 |
| C271/G/R/P/A/V/S/N/Q/D | 34 |
| C176/G/R/P/A/V/S/N/Q/D | 35 |

**Table1C: Further TdT variants at position C173 (SEQ ID NO: 11) or functionally equivalent positions of the indicated SEQ ID NO**

| Substitutions | SEQ ID NO |
|---|---|
| C313/G/R | 10 |
| C173/G/R | 11 |
| C302/G/R | 12 |
| C173/G/R | 13 |
| C302/G/R | 14 |
| C172/G/R | 15 |
| C304/G/R | 16 |
| C173/G/R | 17 |
| C304/G/R | 18 |
| C173/G/R | 19 |
| C293/G/R | 20 |
| C173/G/R | 21 |
| C282/G/R | 22 |
| C178/G/R | 23 |
| C304/G/R | 24 |
| C174/G/R | 25 |
| C300/G/R | 26 |
| C171/G/R | 27 |
| C305/G/R | 28 |
| C173/G/R | 29 |
| C302/G/R | 30 |
| C173/G/R | 31 |
| C313/G/R | 32 |
| C182/G/R | 33 |
| C271/G/R | 34 |
| C176/G/R | 35 |

**Table 2A: Further TdT variants at position C173 (SEQ ID NO: 11) and position M63 (SEQ ID NO: 11) or functionally equivalent positions of the indicated SEQ ID NO**

| Non-wildtype substitutions at locations | SEQ ID NO |
|---|---|
| M63 + C173 | 11 |
| M63 + C173 | 13 |
| L62 + C172 | 15 |
| M63 + C173 | 17 |
| M63 + C173 | 19 |
| R64 + C173 | 21 |
| M73 + C178 | 23 |
| M64 + C174 | 25 |
| M61 + C171 | 27 |
| M63 + C173 | 29 |
| L63 + C173 | 31 |
| M63 + C182 | 33 |
| M66 + C176 | 35 |

**Table 2B: Further TdT variants at position C173 (SEQ ID NO: 11) and position M63 (SEQ ID NO: 11) or functionally equivalent positions of the indicated SEQ ID NO**

| Substitutions and substitution positions | SEQ ID NO |
|---|---|
| M63R/Q/G/A/V/D/N/H/E + C173G/R/P/A/V/S/N/Q/D | 11 |
| M63R/Q/G/A/V/D/N/H/E + C173G/R/P/A/V/S/N/Q/D | 13 |
| L62R/Q/G/A/V/D/N/H/E + C172G/R/P/A/V/S/N/Q/D | 15 |
| M63R/Q/G/A/V/D/N/H/E + C173G/R/P/A/V/S/N/Q/D | 17 |
| M63R/Q/G/A/V/D/N/H/E + C173G/R/P/A/V/S/N/Q/D | 19 |
| R64R/Q/G/A/V/D/N/H/E + C173G/R/P/A/V/S/N/Q/D | 21 |
| M73R/Q/G/A/V/D/N/H/E + C178G/R/P/A/V/S/N/Q/D | 23 |
| M64R/Q/G/A/V/D/N/H/E + C174G/R/P/A/V/S/N/Q/D | 25 |
| M61R/Q/G/A/V/D/N/H/E + C171G/R/P/A/V/S/N/Q/D | 27 |
| M63R/Q/G/A/V/D/N/H/E + C173G/R/P/A/V/S/N/Q/D | 29 |
| L63R/Q/G/A/V/D/N/H/E + C173G/R/P/A/V/S/N/Q/D | 31 |
| M63R/Q/G/A/V/D/N/H/E + C182G/R/P/A/V/S/N/Q/D | 33 |
| M66R/Q/G/A/V/D/N/H/E + C176G/R/P/A/V/S/N/Q/D | 35 |

**Table 2C: Further TdT variants at position C173 (SEQ ID NO: 11) and position M63 (SEQ ID NO: 11) or functionally equivalent positions of the indicated SEQ ID NO**

| Substitutions and substitution positions | SEQ ID NO |
|---|---|
| M63R/Q + C173G/R | 11 |
| M63R/Q + C173G/R | 13 |
| L62R/Q + C172G/R | 15 |
| M63R/Q + C173G/R | 17 |
| M63R/Q + C173G/R | 19 |
| R64R/Q + C173G/R | 21 |
| M73R/Q + C178G/R | 23 |
| M64R/Q + C174G/R | 25 |
| M61R/Q + C171G/R | 27 |
| M63R/Q + C173G/R | 29 |
| L63R/Q + C173G/R | 31 |
| M63R/Q + C182G/R | 33 |
| M66R/Q + C176G/R | 35 |

**Table 3A: Further TdT variants at positions C173 (SEQ ID NO: 11), M63 (SEQ ID NO: 11) and R207 (SEQ ID NO: 11) or functionally equivalent positions of the indicated SEQ ID NO**

| Mutations | SEQ ID NO |
|---|---|
| M63 + C173 + R207 | 11 |
| M63 + C173 + R207 | 13 |
| L62 + C172 + R206 | 15 |
| M63 + C173 + R207 | 17 |
| M63 + C173 + R207 | 19 |
| R64 + C173 + R208 | 21 |
| M73 + C178 + R207 | 23 |
| M64 + C174 + R208 | 25 |
| M61 + C171 + R205 | 27 |
| M63 + C173 + R207 | 29 |
| L63 + C173 + R207 | 31 |
| M63 + C182 + R216 | 33 |
| M66 + C176 + R210 | 35 |

**Table 3B: Further TdT variants at positions C173 (SEQ ID NO: 11), M63 (SEQ ID NO: 11) and R207 (SEQ ID NO: 11) or functionally equivalent positions of the indicated SEQ ID NO**

| Mutations | SEQ ID NO |
|---|---|
| M63R/Q/G/A/V/D/N/H/E + C173G/R/P/A/V/S/N/Q/D + R207 N/L/K/H/G/D/A/P | 11 |
| M63R/Q/G/A/V/D/N/H/E + C173G/R/P/A/V/S/N/Q/D + R207 N/L/K/H/G/D/A/P | 13 |
| L62R/Q/G/A/V/D/N/H/E + C172G/R/P/A/V/S/N/Q/D + R206 N/L/K/H/G/D/A/P | 15 |
| M63R/Q/G/A/V/D/N/H/E + C173G/R/P/A/V/S/N/Q/D + R207 N/L/K/H/G/D/A/P | 17 |
| M63R/Q/G/A/V/D/N/H/E + C173G/R/P/A/V/S/N/Q/D + R207 N/L/K/H/G/D/A/P | 19 |
| R64Q/G/A/V/D/N/H/E + C173G/R/P/A/V/S/N/Q/D + R208 N/L/K/H/G/D/A/P | 21 |
| M73R/Q/G/A/V/D/N/H/E + C178G/R/P/A/V/S/N/Q/D + R207 N/L/K/H/G/D/A/P | 23 |
| M64R/Q/G/A/V/D/N/H/E + C174G/R/P/A/V/S/N/Q/D + R208 N/L/K/H/G/D/A/P | 25 |
| M61R/Q/G/A/V/D/N/H/E + C171G/R/P/A/V/S/N/Q/D + R205 N/L/K/H/G/D/A/P | 27 |
| M63R/Q/G/A/V/D/N/H/E + C173G/R/P/A/V/S/N/Q/D + R207 N/L/K/H/G/D/A/P | 29 |
| L63R/Q/G/A/V/D/N/H/E + C173G/R/P/A/V/S/N/Q/D + R207N/L/K/H/G/D/A/P | 31 |
| M63R/Q/G/A/V/D/N/H/E + C182G/R/P/A/V/S/N/Q/D + R216N/L/K/H/G/D/A/P | 33 |
| M66R/Q/G/A/V/D/N/H/E + C176G/R/P/A/V/S/N/Q/D + R210N/L/K/H/G/D/A/P | 35 |

**Table 3C: Further TdT variants at positions C173 (SEQ ID NO: 11), M63 (SEQ ID NO: 11) and R207 (SEQ ID NO: 11) or functionally equivalent positions of the indicated SEQ ID NO**

| Mutations | SEQ ID NO |
|---|---|
| M63R/Q + C173G/R + R207L/N | 11 |
| M63R/Q + C173G/R + R207L/N | 13 |
| M62R/Q + C172G/R + R206L/N | 15 |
| M63R/Q + C173G/R + R207L/N | 17 |
| M63R/Q + C173G/R + R207L/N | 19 |
| R64Q + C173G/R + R208L/N | 21 |
| M73R/Q + C178G/R + R207N/L | 23 |
| M64R/Q + C 174G/R + R208 N/L | 25 |
| M61R/Q + C171G/R + R205N/L | 27 |
| M63R/Q + C173G/R + R207L/N | 29 |
| L63R/Q + C173G/R + R207L/N | 31 |
| M63R/Q + C182G/R + R216N/L | 33 |
| M66R/Q + C 176G/R + R210N/L | 35 |

**Table 4A: Further TdT variants at positions C173 (SEQ ID NO: 11), M63 (SEQ ID NO: 11), R207 (SEQ ID NO: 11) and R324 (SEQ ID NO: 11) or functionally equivalent positions of the indicated SEQ ID NO**

| Mutations | SEQ ID NO |
|---|---|
| M63 + C173 + R207 + R324 | 11 |
| M63 + C173 + R207 + R324 | 13 |
| L62 + C172 + R206 + R320 | 15 |
| M63 + C173 + R207 + R331 | 17 |
| M63 + C173 + R207 + P325 | 19 |
| R64 + C173 + R208 + T331 | 21 |
| M73 + C178 + R207 + R325 | 23 |
| M64 + C174 + R208 + P326 | 25 |
| M61 + C171 + R205 + R323 | 27 |
| M63 + C173 + R207 + R328 | 29 |
| L63 + C173 + R207 + R325 | 31 |
| M63 + C182 + R216 + R338 | 33 |
| M66 + C176 + R210 + R328 | 35 |

**Table 4B: Further TdT variants at positions C173 (SEQ ID NO: 11), M63 (SEQ ID NO: 11), R207 (SEQ ID NO: 11) and R324 (SEQ ID NO: 11) or functionally equivalent positions of the indicated SEQ ID NO**

| Mutations | SEQ ID NO |
|---|---|
| M63R/Q/G/A/V/D/N/H/E + C173G/R/P/A/V/S/N/Q/D + R207 N/L/K/H/G/D/A/P + R324P/N/A/L/K/H/G/D | 11 |
| M63R/Q/G/A/V/D/N/H/E + C173G/R/P/A/V/S/N/Q/D + R207 N/L/K/H/G/D/A/P + R324P/N/A/L/K/H/G/D | 13 |
| L62R/Q/G/A/V/D/N/H/E + C172G/R/P/A/V/S/N/Q/D + R206 N/L/K/H/G/D/A/P + R320P/N/A/L/K/H/G/D | 15 |
| M63R/Q/G/A/V/D/N/H/E + C173G/R/P/A/V/S/N/Q/D + R207 N/L/K/H/G/D/A/P + R331P/N/A/L/K/H/G/D | 17 |
| M63R/Q/G/A/V/D/N/H/E + C173G/R/P/A/V/S/N/Q/D + R207 N/L/K/H/G/D/A/P + P325N/A/L/K/H/G/D | 19 |
| R64Q/G/A/V/D/N/H/E + C173G/R/P/A/V/S/N/Q/D + R208 N/L/K/H/G/D/A/P + T331P/N/A/L/K/H/G/D | 21 |
| M73R/Q/G/A/V/D/N/H/E + C178G/R/P/A/V/S/N/Q/D + R207 N/L/K/H/G/D/A/P + R325P/N/A/L/K/H/G/D | 23 |
| M64R/Q/G/A/V/D/N/H/E + C174G/R/P/A/V/S/N/Q/D + R208 N/L/K/H/G/D/A/P + P326N/A/L/K/H/G/D | 25 |
| M61R/Q/G/A/V/D/N/H/E + C171G/R/P/A/V/S/N/Q/D + R205 N/L/K/H/G/D/A/P + R323P/N/A/L/K/H/G/D | 27 |
| M63R/Q/G/A/V/D/N/H/E + C173G/R/P/A/V/S/N/Q/D + R207 N/L/K/H/G/D/A/P + R328P/N/A/L/K/H/G/D | 29 |
| L63R/Q/G/A/V/D/N/H/E + C173G/R/P/A/V/S/N/Q/D + R207N/L/K/H/G/D/A/P + R325P/N/A/L/K/H/G/D | 31 |
| M63R/Q/G/A/V/D/N/H/E + C182G/R/P/A/V/S/N/Q/D + R216N/L/K/H/G/D/A/P + R338P/N/A/L/K/H/G/D | 33 |
| M66R/Q/G/A/V/D/N/H/E + C176G/R/P/A/V/S/N/Q/D + R210N/L/K/H/G/D/A/P + R328P/N/A/L/K/H/G/D | 35 |

**Table 4C: Further TdT variants at positions C173 (SEQ ID NO: 11), M63 (SEQ ID NO: 11), R207 (SEQ ID NO: 11) and R324 (SEQ ID NO: 11) or functionally equivalent positions of the indicated SEQ ID NO**

| Mutations | SEQ ID NO |
|---|---|
| M63R/Q + C173G/R + R207N/L + R324P/N | 11 |
| M63R/Q + C173G/R + R207N/L + R324P/N | 13 |
| L62R/Q + C172G/R + R206N/L + R320P/N | 15 |
| M63R/Q + C173G/R + R207N/L + R331P/N | 17 |
| M63R/Q + C173G/R + R207N/L + P325N | 19 |
| R64Q/G + C173G/R + R208N/L + T331P/N | 21 |
| M73R/Q/G + C178G/R + R207N/L + R325P/N | 23 |
| M64R/Q + C174G/R + R208N/L + P326N | 25 |
| M61R/Q + C171G/R + R205N/L + R323P/N | 27 |
| M63R/Q + C173G/R + R207N/L + R328P/N | 29 |
| L63R/Q + C173G/R + R207N/L + R325P/N | 31 |
| M63R/Q + C182G/R + R216N/L + R338P/N | 33 |
| M66R/Q + C176G/R + R210N/L + R328P/N | 35 |

**Table 4D: Further TdT variants at positions C173 (SEQ ID NO: 11), M63 (SEQ ID NO: 11), R207 (SEQ ID NO: 11) and E327 (SEQ ID NO: 11) orfunctionally equivalent positions of the indicated SEQ ID NO**

| Mutations | SEQ ID NO |
|---|---|
| M63 + C173 + R207 + E327 | 11 |
| M63 + C173 + R207 + E327 | 13 |
| L62 + C172 + R206 + G323 | 15 |
| M63 + C173 + R207 + E334 | 17 |
| M63 + C173 + R207 + E327 | 19 |
| R64 + C173 + R208 + E334 | 21 |
| M73 + C178 + R207 + E328 | 23 |
| M64 + C174 + R208 + E329 | 25 |
| M61 + C171 + R205 + E326 | 27 |
| M63 + C173 + R207 + E331 | 29 |
| L63 + C173 + R207 + E328 | 31 |
| M63 + C182 + R216 + E341 | 33 |
| M66 + C176 + R210 + E331 | 35 |

**Table 4E: Further TdT variants at positions C173 (SEQ ID NO: 11), M63 (SEQ ID NO: 11), R207 (SEQ ID NO: 11) and E327 (SEQ ID NO: 11) or functionally equivalent positions of the indicated SEQ ID NO**

| Mutations | SEQ ID NO |
|---|---|
| M63R/Q/G/A/V/D/N/H/E + C173G/R/P/A/V/S/N/Q/D + R207 N/L/K/H/G/D/A/P + E327N/L/T/S | 11 |
| M63R/Q/G/A/V/D/N/H/E + C173G/R/P/A/V/S/N/Q/D + R207 N/L/K/H/G/D/A/P + E327N/L/T/S | 13 |
| L62R/Q/G/A/V/D/N/H/E + C172G/R/P/A/V/S/N/Q/D + R206 N/L/K/H/G/D/A/P + G323N/L/T/S | 15 |
| M63R/Q/G/A/V/D/N/H/E + C173G/R/P/A/V/S/N/Q/D + R207 N/L/K/H/G/D/A/P + E334N/L/T/S | 17 |
| M63R/Q/G/A/V/D/N/H/E + C173G/R/P/A/V/S/N/Q/D + R207 N/L/K/H/G/D/A/P + E327N/L/T/S | 19 |
| R64Q/G/A/V/D/N/H/E + C173G/R/P/A/V/S/N/Q/D + R208 N/L/K/H/G/D/A/P + E334N/L/T/S | 21 |
| M73R/Q/G/A/V/D/N/H/E + C178G/R/P/A/V/S/N/Q/D + R207 N/L/K/H/G/D/A/P + E328N/L/T/S | 23 |
| M64R/Q/G/A/V/D/N/H/E + C174G/R/P/A/V/S/N/Q/D + R208 N/L/K/H/G/D/A/P + E329N/L/T/S | 25 |
| M61R/Q/G/A/V/D/N/H/E + C171G/R/P/A/V/S/N/Q/D + R205 N/L/K/H/G/D/A/P + E326N/L/T/S | 27 |
| M63R/Q/G/A/V/D/N/H/E + C173G/R/P/A/V/S/N/Q/D + R207 N/L/K/H/G/D/A/P + E331N/L/T/S | 29 |
| L63R/Q/G/A/V/D/N/H/E + C173G/R/P/A/V/S/N/Q/D + R207N/L/K/H/G/D/A/P + E328N/L/T/S | 31 |
| M63R/Q/G/A/V/D/N/H/E + C182G/R/P/A/V/S/N/Q/D + R216N/L/K/H/G/D/A/P + E341N/L/T/S | 33 |
| M66R/Q/G/A/V/D/N/H/E + C176G/R/P/A/V/S/N/Q/D + R210N/L/K/H/G/D/A/P + E331N/L/T/S | 35 |

**Table 4F: Further TdT variants at positions C173 (SEQ ID NO: 11), M63 (SEQ ID NO: 11), R207 (SEQ ID NO: 11) and E327 (SEQ ID NO: 11) or functionally equivalent positions of the indicated SEQ ID NO**

| Mutations | SEQ ID NO |
|---|---|
| M63R/Q + C173G/R + R207 N/L + E327N/L/T/S | 11 |
| M63R/Q + C173G/R + R207N/L + E327N/L/T/S | 13 |
| L62R/Q + C172G/R + R206N/L + G323N/L/T/S | 15 |
| M63R/Q + C173G/R + R207N/L + E334N/L/T/S | 17 |
| M63R/Q + C173G/R + R207N/L + E327N/L/T/S | 19 |
| R64Q/G + C173G/R + R208N/L + E334N/L/T/S | 21 |
| M73R/Q + C178G/R + R207N/L + E328N/L/T/S | 23 |
| M64R/Q + C174G/R + R208N/L + E329N/L/T/S | 25 |
| M61R/Q + C171G/R + R205N/L + E326N/L/T/S | 27 |
| M63R/Q/G + C173G/R + R207N/L + E331N/L/T/S | 29 |
| L63R/Q + C173G/R + R207N/L + E328N/L/T/S | 31 |
| M63R/Q + C182G/R + R216N/L + E341N/L/T/S | 33 |
| M66R/Q + C176G/R + R210N/L + E331N/L/T/S | 35 |

The substitution is selected from CzzzG/R/P/A/V/S/N/Q/D, where Czzz represents an amino acid residue number functionally equivalent to C173 of SEQ ID NO: 11 in SEQ ID NOs 13, 15, 17, 19, 21, 23, 25, 27, 29, 31, 33 and 35, respectively, and preferably from CzzzG/R, where Czzz represents an amino acid residue number functionally equivalent to C173 of SEQ ID NO: 11 in SEQ ID NOs 13, 15, 17, 19, 21, 23, 25, 27, 29, 31, 33 and 35, respectively.

The variant further comprises at least one amino acid substitution at position corresponding to functionally equivalent residues of residues selected from M63, R207, R324 and E327, of SEQ ID NO:11.

According to the invention, all variants of TdT as disclosed above are able to both synthesize a nucleic acid fragment without template and incorporate a modified nucleotide into the nucleic acid fragment. Advantageously, said variants have an increased ability to incorporate a modified nucleotide, preferably a 3'O-modified nucleotide, into a nucleic acid fragment as compared to a TdT of SEQ ID NOs : 11, 13, 15, 17, 19, 21, 23, 25, 27, 29, 31, 33 and 35. It is to be emphasized that the scope of the invention is defined by the claims only.

In some of the embodiments described above, the efficiency of a variant TdT in incorporating a 3'O-modified nucleoside triphosphate is at least 110 percent that of a wild type TdT of sequence SEQ ID NO: 11, 13, 15, 17, 19, 21, 23, 25, 27, 29, 31, 33 and 35 in other embodiments, the efficiency of a variant TdT in incorporating a 3'O-modified nucleoside triphosphate is at least 150 percent that of a wild type TdT of sequence SEQ ID NO: 11, 13, 15, 17, 19, 21, 23, 25, 27, 29, 31, 33 and 35; in other embodiments, the efficiency of a variant TdT in incorporating a 3'O-modified nucleoside triphosphate is at least 200 percent that of a wild type TdT of sequence SEQ ID NO: 11, 13, 15, 17, 19, 21, 23, 25, 27, 29, 31, 33 and 35.

The variants of the invention comprise the amino acid sequence of SEQ ID NOs 11, 13, 15, 17, 19, 21, 23, 25, 27, 29, 31, 33 or 35and optionally additional amino acid fragments at the C-ter or N-ter. In another embodiment, the variants of the invention consist solely on the amino acid sequence of SEQ ID NOs 11, 13, 15, 17, 19, 21, 23, 25, 27, 29, 31, 33 or 35.

The variant of Terminal deoxynucleotidyl Transferase (TdT) comprises an amino acid sequence as set forth in SEQ ID NOs 11, 13, 15, 17, 19, 21, 23, 25, 27, 29, 31, 33 or 35 or an amino acid sequence having 90% sequence identity, preferably 95%, 96%, 97%, 98%, 99% sequence identity with any of the foregoing sequences, with at least three amino acid substitutions selected from M63R/Q, L131P, C173G/R, R207L/N, D250V, R324P/N and E327N/L/T/S, or a functionally equivalent residue, wherein the positions are numbered by reference to the amino acid sequence set forth in SEQ ID NO:11 or as set forth directly elsewhere herein in respect of their individual SEQ ID NOs, (i) is able to synthesize a nucleic acid fragment without template and (ii) is able to incorporate a 3'-O-modified nucleotide into the nucleic fragment.

For instance, the variant of TdT comprises an amino acid sequence within a specified percent sequence identity of SEQ ID NO:11, 13, 15, 17, 19, 21, 23, 25, 27, 29, 31, 33 and 35 and a combination of substitutions selected from M63R + L131P + R207L, M63R + L131P + R207N, M63R + L131P + D250V, M63R + L131P + R324P, M63R + L131P + R324A, M63R + L131P + E327L, M63R + L131P + E327N, M63R + R207L + D250V, M63R + R207L + R324P, M63R + R207L + R324A, M63R + R207L + E327L, M63R + R207L + E327N, M63R + R207N + D250V, M63R + R207N + R324P, M63R + R207N + R324A, M63R + R207N + E327L, M63R + R207N + E327N, M63R + D250V + R324P, M63R + D250V + R324A, M63R + R324P + E327L, M63R + R324P + E327N, M63R + R324A + E327L, M63R + R324A + E327N, M63Q + L131P + R207L, M63Q + L131P + R207N, M63Q + L131P + D250V, M63Q + L131P + R324P, M63Q + L131P + R324A, M63Q + L131P + E327L, M63Q + L131P + E327N, M63Q + R207L + D250V, M63Q + R207L + R324P, M63Q + R207L + R324A, M63Q + R207L + E327L, M63Q + R207L + E327N, M63Q + D250V + R324P, M63Q + D250V + R324A, M63Q + D250V + E327L, M63Q + D250V + E327N, M63Q + R324P + E327L, M63Q + R324P + E327N, M63Q + R324A + E327L, M63Q + R324A + E327N, L131P + R207L + D250V, L131P + R207L + R324A, L131P + R207L + E327L, L131P + R207L + E327N, L131P + R207N + D250V, L131P + R207N + R324P, L131P + R207N + R324A, L131P + R207N + E327L, L131P + R207N + E327N, L131P + D250V + R324P, L131P + D250V + R324A, L131P + D250V + E327L, L131P + D250V + E327N, L131P + R324P + E327L, L131P + R324P + E327N, L131P + R324A + E327L, L131P + R324A + E327N, R207L + D250V + R324P, R207L + D250V + R324A, R207L + D250V + E327L, R207L + D250V + E327N, R207L + R324P + E327L, R207L + R324P + E327N, R207L + R324A + E327L, R207L + R324A + E327N, R207N + D250V + R324P, R207N + D250V + R324A, R207N + D250V + E327L, R207N + D250V + E327N, R207N + R324P + E327L, R207N + R324P + E327N, R207N + R324A + E327L, R207N + R324A + E327N, D250V + R324P + E327L, D250V + R324P + E327N, D250V + R324A + E327L, D250V + R324A + E327N and R207L + D250V + R324P, or functionally equivalent residue(s) wherein the above position numbers are with respect to SEQ ID NO 11.

The variant of TdT comprises an amino acid sequence within a specified percent sequence identity of SEQ ID N°11, or functionally equivalent sequence, with the combination of substitutions R207L + R324P + E327L, or functionally equivalent residues.

The variant of TdT comprises an amino acid sequence within a specified percent sequence identity of SEQ ID N°11, or functionally equivalent sequence, with the combination of substitutions R207N + R324A + E327N, or functionally equivalent residues. Such variant may further comprise at least one substitution at position corresponding to residues selected from L52, A108, L131, T340, G284, H287, E289, W450, R354 and A510, with respect to SEQ ID NO 11, or functionally equivalent residue(s) in functionally equivalent sequence. As exposed above, said variant may also comprise the combination of constant mutations L52F+A108V+R354K and/or G284L/S+H287D+E289A, with respect to SEQ ID NO:11, or functionally equivalent residue(s) in functionally equivalent sequence.

The disclosure
provides a variant of Terminal deoxynucleotidyl Transferase (TdT) which comprises an amino acid sequence within a specified percent sequence identity of SEQ ID NO:11 or a functionally equivalent sequence, with at least one amino acid substitution selected from M63R, M63Q, L131P, R207L, R207N, D250V, R324P, R324A, E327L, E327N, or functionally equivalent residue(s), (i) is able to synthesize a nucleic acid fragment without a template and (ii) is able to incorporate a 3'-O-modified nucleotide into the nucleic fragment.

The disclosure provides a variant of Terminal deoxynucleotidyl Transferase (TdT) which comprises an amino acid sequence within a specified percent sequence identity of SEQ ID NO:11 or a functionally equivalent sequence, with at least the combination of substitutions selected from M63R + L131P, M63R + R207L, M63R + R207N, M63R + D250V, M63R + R324P, M63R + R324A, M63R + E327L, M63R + E327N, M63Q + L131P, M63Q + R207L, M63Q + R207N, M63Q + D250V, M63Q + R324P, M63Q + R324A, M63Q + E327L, M63Q + E327N, L131P + R207L, L131P + R207N, L131P + D250V, L131P + R324P, L131P + R324A, L131P + E327L, L131P + E327N, R207L + D250V, R207L + R324P, R207L + R324A, R207L + E327L, R207L + E327N, R207N + D250V, R207N + R324P, R207N + R324A, R207N + E327L, R207N + E327N, D250V + R324P, D250V + R324A, D250V + E327L, D250V + E327N, R324P + E327L, R324P + E327N, R324A + E327L and R324A + E327N, or functionally equivalent residue(s), wherein the positions are numbered by reference to the amino acid sequence set forth in SEQ ID N°11, (i) is able to synthesize a nucleic acid fragment without a template and (ii) is able to incorporate a 3'-O-modified nucleotide into the nucleic fragment.

A variant of TdT has a substitution or combination of substitutions described above and has an amino acid sequence within at least 80% identity with SEQ ID NOs 11, 13, 15, 17, 19, 21, 23, 25, 27, 29, 31, 33 or 35; in some embodiments, such amino acid sequence is within at least 85%, 90%, 95%, 96%, 97%, 98%, or 99% identity with SEQ ID NOs 11, 13, 15, 17, 19, 21, 23, 25, 27, 29, 31, 33 or 35.

### Additional modifications

In an embodiment, the variant of TdT further includes any type of tagging peptide in its N-terminal, C-terminal or both extremity, such as a His-tag sequence. Said tagging peptide could be used for purification, identification, increasing expression, secretability or increasing catalytic activity. It will be understood that such different tags are extensively described in the literature and thus all tag known to a skilled person are covered by the present invention.

The variants of the invention can also include one or more exogenous or heterologous features at the N- and/or C-terminal regions of the protein for use, e.g., in the purification of the recombinant polymerase.

The variant of the invention may further comprise a substitution of residues between positions C378 to L406, wherein the positions are numbered by reference to the amino acid sequence set forth in SEQ ID N°1, or functionally equivalent residues, by residues H363 to C390 of the Polµ polymerase of sequence SEQ ID NO:3, wherein the positions are numbered by reference to the amino acid sequence set forth in SEQ ID N°3 or functionally equivalent residues.

Advantageously, the variant of TdT comprises at least the amino acid sequence SEQ ID NO: 11, 13, 15, 17, 19, 21, 23, 25, 27, 29, 31, 33 or 35, with the disclosed substitution(s) and percent sequence identity values.

### Nucleic acids, expression cassette, vector

It is also the purpose of the invention to provide a nucleic acid molecule encoding a variant of the invention. As used herein, the term *"nucleic acid", "nucleic sequence," "polynucleotide", "oligonucleotide"* and *"nucleotide sequence"* are used interchangeably and refer to a sequence of deoxyribonucleotides and/or ribonucleotides. In one embodiment, the nucleic acid is a DNA. In an alternative embodiment, the nucleic acid is RNA. In an alternative embodiment, the nucleic acid is XNA.

The nucleic acids can be in single stranded form or in duplex form or a mixture of the two. It can be of recombinant, artificial and/or synthetic origin and it can comprise modified nucleotides. Such modifications could be natural modifications such as epigenetic modifications, or unnatural modification such as labels, modified bond, a modified purine or pyrimidine base, or a modified sugar. In one embodiment, nucleic acid molecules are DNA, RNA or XNA bearing naturally occurring epigenetic modifications such as methylation, hydfroxymethylation, formylation or 5-carboxylation. In one embodiment, nucleic acid molecules are DNA, RNA or XNA bearing unnaturally occurring modifications such as fluorescent tag, fluorescent label, interaction groups.

The nucleic acids of the invention can be in isolated or purified form, and made, isolated and/or manipulated by techniques known per se in the art, e.g., cloning and expression of cDNA libraries, amplification, enzymatic synthesis or recombinant technology. The nucleic acids can also be synthesized in vitro by well-known chemical synthesis techniques, as described in, e.g., Belousov (1997) Nucleic Acids Res. 25:3440-3444.

The invention also encompasses nucleic acids which hybridize, under stringent conditions, to a nucleic acid encoding a TdT variant as defined above. Preferably, such stringent conditions include incubations of hybridization filters at about 42° C for about 2.5 hours in 2 X SSC/0.1%SDS, followed by washing of the filters four times of 15 minutes in 1 X SSC/0.1% SDS at 65° C. Protocols used are described in such reference as Sambrook et al. (Molecular Cloning: a Laboratory Manual, Cold Spring Harbor Press, Cold Spring Harbor N.Y. (1988)) and Ausubel (Current Protocols in Molecular Biology (1989)).

The invention also encompasses nucleic acids encoding a TdT variant of the invention, wherein the sequence of said nucleic acids, or a portion of said sequence at least, has been engineered using optimized codon usage.

Alternatively, the nucleic acids according to the invention may be deduced from the sequence of the TdT variant according to the invention and codon usage may be adapted according to the host cell in which the nucleic acids shall be transcribed. These steps may be carried out according to methods well known to one skilled in the art and some of which are described in the reference manual Sambrook et al. (Sambrook et al., 2001).

In one embodiment, nucleic acid molecules are polymeric molecules having length of more than 50, 100, 200, 300, 400, 500, 600, 700, 800, 900, 1 000, 2 000, 3 000, 4 000, 5 000, 6 000, 7 000, 8 000, 9 000, 10 000, 15 000, 20 000, 30 000, 40 000, 50 000 or 100 000 nucleotides.

Nucleic acids of the invention may further comprise additional nucleotide sequences, such as regulatory regions, i.e., promoters, enhancers, silencers, terminators, signal peptides and the like that can be used to cause or regulate expression of the polypeptide in a selected host cell or system.

The present invention further relates to an expression cassette comprising a nucleic acid according to the invention operably linked to one or more control sequences that direct the expression of said nucleic acid in a suitable host cell. Typically, the expression cassette comprises, or consists of, a nucleic acid according to the invention operably linked to a control sequence such as transcriptional promoter and/or transcription terminator. The control sequence may include a promoter that is recognized by a host cell or an *in vitro* expression system for expression of a nucleic acid encoding a TdT variant of the present invention. The promoter contains transcriptional control sequences that mediate the expression of the enzyme. The promoter may be any polynucleotide that shows transcriptional activity in the host cell including mutant, truncated, and hybrid promoters, and may be obtained from genes encoding extracellular or intracellular polypeptides either homologous or heterologous to the host cell. The control sequence may also be a transcription terminator, which is recognized by a host cell to terminate transcription. The terminator is operably linked to the 3'-terminus of the nucleic acid encoding the esterase. Any terminator that is functional in the host cell may be used in the present invention. Typically, the expression cassette comprises, or consists of, a nucleic acid according to the invention operably linked to a transcriptional promoter and a transcription terminator.

The invention also relates to a vector comprising a nucleic acid or an expression cassette as defined above.

The term *"vector"* refers to DNA molecule used as a vehicle to transfer recombinant genetic material into a host cell. The major types of vectors are plasmids, bacteriophages, viruses, cosmids, and artificial chromosomes. The vector itself is generally a DNA sequence that consists of an insert (a heterologous nucleic acid sequence, transgene) and a larger sequence that serves as the "backbone" of the vector. The purpose of a vector which transfers genetic information to the host is typically to isolate, multiply, or express the insert in the target cell. Vectors called expression vectors (expression constructs) are specifically adapted for the expression of the heterologous sequences in the target cell, and generally have a promoter sequence that drives expression of the heterologous sequences encoding a polypeptide. Generally, the regulatory elements that are present in an expression vector include a transcriptional promoter, a ribosome binding site, a terminator, and optionally present operator. Preferably, an expression vector also contains an origin of replication for autonomous replication in a host cell, a selectable marker, a limited number of useful restriction enzyme sites, and a potential for high copy number. Examples of expression vectors are cloning vectors, modified cloning vectors, specifically designed plasmids and viruses. Expression vectors providing suitable levels of polypeptide expression in different hosts are well known in the art. The choice of the vector will typically depend on the compatibility of the vector with the host cell into which the vector is to be introduced.

It is another object of the invention to provide a host cell comprising a nucleic acid, an expression cassette or a vector as described above. The present invention thus relates to the use of a nucleic acid, expression cassette or vector according to the invention to transform, transfect or transduce a host cell. The choice of the vector will typically depend on the compatibility of the vector with the host cell into which it must be introduced.

According to the invention, the host cell may be transformed, transfected or transduced in a transient or stable manner. The expression cassette or vector of the invention is introduced into a host cell so that the cassette or vector is maintained as a chromosomal integrant or as a self-replicating extra-chromosomal vector. The term "host cell" also encompasses any progeny of a parent host cell that is not identical to the parent host cell due to mutations that occur during replication. The host cell may be any cell useful in the production of a variant of the present invention, e.g., a prokaryote or a eukaryote. The prokaryotic host cell may be any Gram-positive or Gram-negative bacterium. The host cell may also be an eukaryotic cell, such as a yeast, fungal, mammalian, insect or plant cell.

The nucleic acid, expression cassette or expression vector according to the invention may be introduced into the host cell by any method known by the skilled person, such as electroporation, conjugation, transduction, competent cell transformation, protoplast transformation, protoplast fusion, biolistic "gene gun" transformation, PEG-mediated transformation, lipid-assisted transformation or transfection, chemically mediated transfection, lithium acetate-mediated transformation, liposome-mediated transformation,

Optionally, more than one copy of a nucleic acid, cassette or vector of the present invention may be inserted into a host cell to increase production of the variant.

### Modified nucleotides

According to the invention, the variants of TdT are able to incorporate modified nucleotides, preferably modified 3'O- nucleotides and more preferably 3'-O-blocked nucleotides.

In the context of the invention, the expression *"Modified Nucleotide"* refers to a molecule containing a nucleoside (i.e. a base attached to a deoxyribose or ribose sugar molecule) bound to three phosphate groups which has at least one additional group on one of its extremity: 2', 3', 5' or base. Said additional group blocks further addition of nucleotides by preventing the formation of any phosphodiester bond (3'O-modification, 2' or 2'O modifications) or by sterically preventing the polymerase to attach to any nucleic acid fragments that comprises on its 3' extremity such modified nucleotide (5' or base modification). Furtherly, said additional group has advantagesoulsy a reversible nature allowing that group to be removed through a specific cleaving reaction.

Nucleosides or nucleotide triphosphates include deoxyadenosine triphosphate (dATP), deoxyguanosine triphosphate (dGTP), deoxycytidine triphosphate (dCTP) or deoxythymidine triphosphate (dTTP) for examples of nucleotide containing deoxyribose. Adenosine triphosphate (ATP), guanosine triphosphate (GTP), cytidine triphosphate (CTP) or uridine triphosphate (UTP) are further examples of nucleotide triphosphates containing ribose. Other types of nucleosides may be bound to three phosphates to form nucleotide triphosphates, such as naturally occurring modified nucleosides and artificial nucleosides.

In a particular embodiment, the modified nucleotide is a 3'O-blocked nucleotide, which comprises a group reversibly attached to the 3' end of the nucleotide triphosphate to prevent further nucleotide addition. Said group could have diverse chemical natures, such as azidomethyl, aminoxy, and allyl.

Advantageously, the modified nucleotide is selected from a 3'-O-NH₂-nucleoside triphosphate, a 3'-O-azidomethyl-nucleoside triphosphate, a 3'-O-allyl-nucleoside triphosphate, a 3'O-(2-nitrobenzyl)-nucleoside triphosphate, or a 3'-O-propargyl-nucleoside triphosphate.

In some embodiments, the modified nucleotides comprise a modified nucleotide or nucleoside molecule comprising a purine or pyrimidine base and a ribose or deoxyribose sugar moiety having a removable 3'-OH blocking group covalently attached thereto, such that the 3' carbon atom has attached a group of the structure:

-O-Z

wherein -Z is any of -C(R')2-0-R", -C(R')2-N(R")2, -C(R')2-N(H)R", -C(R')2-S-R" and - C(R')2-F, wherein each R" is or is part of a removable protecting group; each R' is independently a hydrogen atom, an alkyl, substituted alkyl, arylalkyl, alkenyl, alkynyl, aryl, heteroaryl, heterocyclic, acyl, cyano, alkoxy, aryloxy, heteroaryloxy or amido group, or a detectable label attached through a linking group; with the proviso that in some embodiments such substituents have up to 10 carbon atoms and/or up to 5 oxygen or nitrogen heteroatoms; or (R')2 represents an alkylidene group of formula =C(R‴)2 wherein each R‴ may be the same or different and is selected from the group comprising hydrogen and halogen atoms and alkyl groups, with the proviso that in some embodiments the alkyl of each R‴ has from 1 to 3 carbon atoms; and wherein the molecule may be reacted to yield an intermediate in which each R" is exchanged for H or, where Z is -(R')2-F, the F is exchanged for OH, SH or NH2, preferably OH, which intermediate dissociates under aqueous conditions to afford a molecule with a free 3'-OH; with the proviso that where Z is -C(R')2-S-R", both R' groups are not H. In certain embodiments, R' of the modified nucleotide or nucleoside is an alkyl or substituted alkyl, with the proviso that such alkyl or substituted alkyl has from 1 to 10 carbon atoms and from 0 to 4 oxygen or nitrogen heteroatoms. In certain embodiments, -Z of the modified nucleotide or nucleoside is of formula -C(R')₂-N3. In certain embodiments, Z is an azidomethyl group.

In some embodiments, Z is a cleavable organic moiety with or without heteroatoms having a molecular weight of 200 or less. In other embodiments, Z is a cleavable organic moiety with or without heteroatoms having a molecular weight of 100 or less. In other embodiments, Z is a cleavable organic moiety with or without heteroatoms having a molecular weight of 50 or less.

In a further particular embodiment, "3'O modified nucleotide" refers to nucleotide triphosphate bearing at the 3' extremity either a 3'-O-methyl, 3'-azido, 3'-O-azidomethyl, 3'-O-amino, 3'-aminoxy or 3'-O-allyl group. In a further embodiment, the 3'-blocked nucleotide triphosphate is blocked by either a 3'-O-azidomethyl, 3'-aminoxy or 3'-O-allyl group. In other embodiments, "3'O modified nucleotide" refers to nucleotide triphosphate bearing at the 3' extremity either esters, ethers, carbonitriles, phosphates, carbonates, carbamates, hydroxylamine, borates, nitrates, sugars, phosphoramide, phosphoramidates, phenylsulfenates, sulfates, sulfones or amino acids. In some embodiments, the foregoing 3'-O-blocking groups have a molecule weight of 100 or less.

In another embodiments, 3'-O-blocking groups of the invention include methyl, 3'-O-(2-nitrobenzyl), allyl, amine, azidomethyl, tert-butoxy ethoxy, or propargyl.

In further particular embodiment, "3'O modified nucleotide" refers to a nucleotide triphosphate having a terminator effector modifying group such as those described in WO2016034807.

Interestingly, the variants of the invention exhibit an increased affinity for modified nucleotides, as compared to wild type TdT, and thereby an increased ability to incorporate such modified nucleotide in a nucleic acid sequence during nucleic acid synthesis. More particularly, the variants of the invention are able to use and incorporate modified 3'O- nucleotides (and more particularly, 3'O-blocked nucleotide) in nucleic acid sequence, which is not possible with wild type TdT (see Knapp et al. Chem. Eur. J., 2011, 17:2903).

According to a particular aspect, the invention relates to variants of TdT able to work with modified nucleotides in a nucleic acids enzymatic synthesis process, particularly with 3'O-modified nucleotides (e.g., 3'O-blocked nucleotide), and having the ability to produce long length nucleic acid molecules or derivative of nucleic acid molecules.

### Enzymatic Synthesis of nucleic acid

It is the purpose of the present invention to provide variants of TdT that may be used for the synthesis of nucleic acid, such as described in Ybert et al, WO2015/159023; Jensen et al, Biochemistry, 57: 1821-1832 (2018); Hiatt et al, U.S. patent 5808045. More particularly, it is the purpose of the present invention to provide variants of TdT suitable to add modified nucleotides to an initiating nucleic acid strand. The blocking group may be then removed for allowing a new addition of modified nucleotide.

According to the invention, by use of a variant of the invention, it is possible to implement successive cycles comprising additions and deprotections. This process will therefore allow by multiple cycles of addition of a reversible modified nucleotide and further removal of the blocking group to allow the controlled extension of an initiating nucleic acid strand into a defined sequence.

The present invention contemplates the use of modified TdT according to the present invention in any enzymatic nucleic acid synthesis process.

It is thus an object of the present invention to provide a method of synthesizing a polynucleotide having a predetermined sequence, comprising the steps of:
a) providing an initiator having a 3'-terminal nucleotide having a free 3'-hydroxyl;
b) repeating cycles of (i) contacting under elongation conditions the initiator or elongated fragments having free 3'-O-hydroxyls with a 3'-O-blocked nucleoside triphosphate and a TdT variant of the present invention, so that the initiator or elongated fragments are elongated by incorporation of a 3'-O-blocked nucleoside triphosphate to form 3'-O-blocked elongated fragments, and (ii) deblocking the elongated fragments to form elongated fragments having free 3'-hydroxyls, until the polynucleotide is formed.

It is also the purpose of the present invention to provide a process for synthesizing a nucleic acid molecule without template, comprising a step of contacting a nucleic acid primer with both at least one nucleotide, preferably at least one 3'O-modified nucleotide, and a variant of the invention.

The present invention contemplates the concept of enzymatic nucleic acids synthesis process. In such process, nucleic acids molecules are *de novo* synthesized in absence of any template strand. Accordingly, ordered sequence of nucleotides are coupled to an initiator nucleic acid fragment with the help of the variant of the invention. It will be understood that quantitative coupling and more generally high coupling efficiency of each nucleotide to the growing nucleic acid chain is of great importance. It will also be understood that non-terminator nucleotides, such as natural nucleotides or permanent labeled nucleotides, will not permit any control over the sequence synthesized and will result, for example, in uncontrolled and undesired poly-additions.

In some embodiments, the method of synthesizing a polynucleotide comprises the steps of (a) providing an initiator having a free 3'-hydroxyl; (b) reacting under extension conditions the initiator or an extension intermediate having a free 3'-hydroxyl with a variant TdT of the invention in the presence of a 3'-O-blocked nucleoside triphosphate to produce a 3'-O-blocked extension intermediate; (c) deblocking the extension intermediate to produce an extension intermediate with a free 3'-hydroxyl; and (d) repeating steps (b) and (c) until the polynucleotide is synthesized.

In some embodiments, the method of synthesizing a polynucleotide comprises the steps of (a) providing an initiator attached to a solid support, the iniator being an oligonucleotide having a free 3'-hydroxyl; (b) reacting under extension conditions the initiator or an extension intermediate having a free 3'-hydroxyl with a variant TdT of the invention in the presence of a 3'-O-blocked nucleoside triphosphate to produce a 3'-O-blocked extension intermediate; (c) washing the solid support to remove unincorporated 3'-O-blocked nucleoside triphosphate; (d) deblocking the extension intermediate by exposing the solid support to a deblocking agent to produce an extension intermediate having a free 3'-hydroxyl; and (e) repeating steps (b) and (d) until the polynucleotide is synthesized. The method may include a further step of cleaving the completed polynucleotide from the solid support.

In some embodiments, for TdT catalyzed addition reactions, the enzymatic conditions may contain from about 0.20 and about 200 µM of the nucleotide having the removable blocking moiety protecting the 3'-hydroxyl and from about 0.20 to 200 µM of free and unmodified 3'-hydroxyls derived from the initiating substrate. In some embodiments, the reaction buffer contains from about 10 to about 500 mM potassium cacodylate buffer (pH between 6.5 and 7.5). and from about 0.01 to about 10 mM of a divalent cation (e.g. CoCl₂ or MnCl₂). Other buffer compositions and components may be suitable for particular desired embodiment of the present invention.

In the context of the invention, the expression "cleaving reaction" refers to any action of substance or physical conditions, which is able to cleave the additional group previously described on reversible modified nucleotides. A person skilled in the art is able to determine a cleaving reaction for any previously listed group.

In one embodiment, the cleaving agent is a chemical cleaving agent. In an alternative embodiment, the cleaving agent is an enzymatic cleaving agent.

It will be understood by the person skilled in the art that the selection of cleaving agent is dependent on the type of 3'-nucleotide blocking group used. For example, tris(2-carboxyethyl)phosphine (TCEP) can be used to cleave a 3'O-azidomethyl groups, palladium complexes can be used to cleave a 3'O-allyl groups, or sodium nitrite can be used to cleave a 3'O-amino group. In particular embodiment, the cleaving reaction is involving: TCEP, a palladium complex or sodium nitrite.

In particular embodiment, the cleaving reaction is performed in the presence of additional components such as denaturant (urea, guanidinium chloride, formamide or betaine for example). In a further embodiment, the cleavage reaction is performed with one or more buffers. It will be understood by the person skilled in the art that the choice of buffer is dependent on the exact mechanism of reaction.

The present invention relates to variants of TdT with the capacity to incorporate, in a quantitative way, modified nucleotides. By "quantitative way" or "quantitative reaction", it is meant a reaction that goes to completion, i.e. in which reactants are totally converted into the product. Polymerase that incorporates in a quantitative way reversible modified nucleotide is a polymerase able to elongate every fragment of nucleic acid with all the nucleotides available leading to the conversion of all the initiating fragments of length n, to fragments of length n+1.

As used herein, "initiating fragment" refers to a short oligonucleotide sequence with a free 3'-end, which can be further elongated. In one embodiment, the initiating fragment is a DNA initiating fragment. In an alternative embodiment, the initiating fragment is an RNA initiating fragment.

In one embodiment, the initiating fragment possesses between 3 and 100 nucleotides, in particular between 3 and 20 nucleotides.

In one embodiment, the initiating fragment is single-stranded. In an alternative embodiment, the initiating fragment is double-stranded.

In one embodiment, the initiating fragment is immobilized on a solid support. The initiating fragment may be attached with various method to a solid support resulting in a stable under the various enzymatic or synthesis reaction conditions that the fragment will undergo.

In one embodiment, the initiating fragment is immobilized on a solid support via a reversible interacting moiety, such as a chemically-cleavable linker, an antibody/immunogenic epitope, a biotin/biotin-binding protein or glutathione-GST tag. In a further embodiment, the initiating fragment is immobilized on a solid support via a chemically-cleavable linker, such as a disulfide, allyl, or azide-masked hemiaminal ether linker.

In an initiating fragment, the immobilized part contains at least one restriction site. The use of restriction enzymes and restriction sites to selectively hydrolyze nucleic acids chain at a specific site is describe in the literature. Any skilled person will be able to choose the appropriate restriction enzyme that will match the initiating fragment cleaving site sequence.

In an alternative embodiment, the initiating fragment contains at least one uridine. Treatment with uracil-DNA glycosylase (UDG) generates an abasic site. Treatment on an appropriate substrate with an apurinic/apyrimidinic (AP) site endonuclease will extract the nucleic acid strand.

### Applications

Described herein is the use of variants of TdT to be used for nucleic acid synthesis, oligonucleotide synthesis, probe synthesis, tagging, nucleic acid amplification, aptamers, therapeutic nucleic acid molecules, drug target discovery and validation, disease diagnosis, metabolic engineering, data storage, crops improvement, library design, sequencing pools, nucleic acid labeling or attachment or any other application that is involving nucleic acid molecules.

### Production of Variant TdTs

Variants of the invention may be produced by mutating known reference or wild type TdT-coding polynucleotides, then expressing it using conventional molecular biology techniques. For example, the mouse TdT gene (SEQ ID NO: 1) may be assembled from synthetic fragments using conventional molecular biology techniques, e.g. using protocols described by Stemmer et al, Gene, 164: 49-53 (1995); Kodumal et al, Proc. Natl. Acad. Sci., 101: 15573-15578 (2004); or the like, or it may be directly cloned from mouse cells using protocols described by Boule et al, Mol. Biotechnology, 10: 199-208 (1998), or Bentolila et al, EMBO J., 14: 4221-4229 (1995); or the like.

For example, an isolated TdT gene may be inserted into an expression vector, such as pET32 (Novagen) to give a vector pCTdT which then may be used to make and express variant TdT proteins using conventional protocols. Vectors with the correct sequence may be transformed in E. coli producer strains.

Transformed strains are cultured using conventional techniques to pellets from which TdT protein is extracted. For example, previously prepared pellets are thawed in 30 to 37°C water bath. Once fully thawed, pellets are resuspended in lysis buffer composed of 50mM tris-HCL (Sigma) pH 7.5, 150mM NaCl (Sigma), 0.5mM mercaptoethanol (Sigma), 5% glycerol (Sigma), 20mM imidazole (Sigma) and 1 tab for 100mL of protease cocktail inhibitor (Thermofisher). Careful resuspension is carried out in order to avoid premature lysis and remaining of aggregates. Resuspended cells are lysed through several cycles of French press, until full color homogeneity is obtained. Usual pressure used is 14,000psi. Lysate is then centrifuged for 1h to 1h30 at 10,000 rpm. Centrifugate is pass through a 0.2µm filter to remove any debris before column purification.

TdT protein may be purified from the centrifugate in a one-step affinity procedure. For example, Ni-NTA affinity column (GE Healthcare) is used to bind the polymerases. Initially the column has been washed and equilibrated with 15 column volumes of 50mM tris-HCL (Sigma) pH 7.5, 150mM NaCl (Sigma) and 20mM imidazole (Sigma). Polymerases are bound to the column after equilibration. Then a washing buffer, composed of 50mM tris-HCL (Sigma) pH 7.5, 500mM NaCl (Sigma) and 20mM imidazole (Sigma), is applied to the column for 15 column volumes. After wash the polymerases are eluted with 50mM tris-HCL (Sigma) pH 7.5, 500mM NaCl (Sigma) and 0.5M imidazole (Sigma). Fractions corresponding to the highest concentration of polymerases of interest are collected and pooled in a single sample. The pooled fractions are dialyzed against the dialysis buffer (20 mM Tris-HCl, pH 6.8, 200mM Na Cl, 50mM MgOAc, 100mM [NH4]2SO4). The dialysate is subsequently concentrated with the help of concentration filters (Amicon Ultra-30, Merk Millipore). Concentrated enzyme is distributed in small aliquots, 50% glycerol final is added, and those aliquots are then frozen at -20°C and stored for long term. 5µL of various fraction of the purified enzymes are analyzed in SDSPAGE gels.

### Kits, Enzyme and Nucleotide Composition

A particular aspect of the invention is relative to the composition and the use of kits comprising a variant of TdT according to the invention, or to any particular aspect of the present invention, with optionally any combination of one or more components selected from: an initiating fragment, one or more reversible terminator nucleotides, additional enzyme and reagents used in a cleaving reaction. Said kits can be used in a method of enzymatic nucleic acid synthesis.

The present invention covers the composition of matter comprising variants of TdT according to the invention, or to any particular aspect of the present invention, with reversible modified nucleotide in a mix with appropriate buffer and ratio concentration.

### EXAMPLES

### Example 1 - Generation, expression and purification of variants of TdT according to the invention

### Expression strain generation

The TdT mouse gene is been generated from the pET28 plasmid described in [Boulé et al., 1998, Mol. Biotechnol. 10, 199-208]. Sequence SEQ ID NO:4 (Tag TdT) is been amplified by using the following primers:
- T7-pro: TAATACGACTCACTATAGGG (SEQ ID NO:5)
- T7-ter: GCTAGTTATTGCTCAGCGG (SEQ ID NO:6)
through standard molecular biology techniques. The sequence is then cloned into plasmid pET32 backbone to give the new pCTdT plasmid.

After sequencing pCTdT is transformed into commercial *E. coli* cells, BL21 (DE3, from Novagen). Growing colonies on plate with kanamycin are isolated and named Ec-CTdT.

### Polymerase variants generation

The pCTdT vector is used as starting vector. Specific primers comprising one or several point mutations are been generated from Agilent online software (http://www.genomics.agilent.com:80/primerDesignProgram.jsp). The commercially available kit QuickChange II (Agilent) has been used to generate the desired modified polymerase comprising the targeted mutations. Experimental procedure has followed the supplier's protocol. After generation of the different vectors, each of them have been sequenced. Vectors with the correct sequence have been transformed in *E. coli* producer strains, as described before. Clones able to grow on kanamycin LB-agar plates are isolated.

### Expression

The Ec-CTdT and Ec-clone' strains are used for inoculating 250mL erlens with 50mL of LB media supplemented with appropriate amount of kanamycin. After overnight growth at 37°C, appropriate volumes of these pre-cultures are used to inoculate 5L erlens with 2L LB media with kanamycin. The initial OD for the 5L cultures is chosen to be 0.01. The erlens are put at 37°C under strong agitation and the OD of the different cultures are regularly checked. After reaching an OD comprised between 0.6 and 0.9 each erlen is supplemented by the addition of 1mL of 1M IPTG (Isopropyl β-D-1-thiogalactopyranoside, Sigma). The erlens are put back to agitation under a controlled temperature of 37°C. After overnight expression, the cells are harvested in several pellets. Pellets expressing the same variants are pooled and stored at -20°C, eventually for several months.

### Extraction

Previously prepared pellets are thawed in 30 to 37°C water bath. Once fully thawed, pellets are resuspended in lysis buffer composed of 50mM tris-HCL (Sigma) pH 7.5, 150mM NaCl (Sigma), 0.5mM mercaptoethanol (Sigma), 5% glycerol (Sigma), 20mM imidazole (Sigma) and 1 tab for 100mL of protease cocktail inhibitor (Thermofisher). Careful resuspension is carried out in order to avoid premature lysis and remaining of aggregates. Resuspended cells are lysed through several cycles of French press, until full color homogeneity is obtained. Usual pressure used is 14,000psi. Lysate is then centrifuged for 1h to 1h30 at 10,000 rpm. Centrifugate is pass through a 0.2µm filter to remove any debris before column purification.

### Purification

A one-step affinity procedure is used to purify the produced and extracted polymerase enzymes. A Ni-NTA affinity column (GE Healthcare) is used to bind the polymerases. Initially the column has been washed and equilibrated with 15 column volumes of 50mM tris-HCL (Sigma) pH 7.5, 150mM NaCl (Sigma) and 20mM imidazole (Sigma). Polymerases are bound to the column after equilibration. Then a washing buffer, composed of 50mM tris-HCL (Sigma) pH 7.5, 500mM NaCl (Sigma) and 20mM imidazole (Sigma), is applied to the column for 15 column volumes. After wash the polymerases are eluted with 50mM tris-HCL (Sigma) pH 7.5, 500mM NaCl (Sigma) and 0.5M imidazole (Sigma). Fractions corresponding to the highest concentration of polymerases of interest are collected and pooled in a single sample. The pooled fractions are dialyzed against the dialysis buffer (20 mM Tris-HCl, pH 6.8, 200mM Na Cl, 50mM MgOAc, 100mM [NH₄]₂SO₄). The dialysate is subsequently concentrated with the help of concentration filters (Amicon Ultra-30, Merk Millipore). Concentrated enzyme is distributed in small aliquots, 50% glycerol final is added, and those aliquots are then frozen at -20°C and stored for long term. 5µL of various fraction of the purified enzymes are analyzed in SDSPAGE gels.

### Example 2 - Evaluation of the activity of variants of TdT with fluorescent primers

### Activity Test

Elongation performance of TdT variants generated, expressed and purified according to example 1 is evaluated through the following assay. All the results are compared with each other and with the wild type TdT enzyme and to a control tube lacking any polymerase enzyme.

**Table 5: Activity test**

| **Reagent** | **Concentration** | **Volume** |
|---|---|---|
| H₂O | - | 12 µL |
| Activity Buffer | 10x | 2 µL |
| dNTP | 250 µM | 2 µL |
| Purified enzyme | 20 µM | 2 µL |
| Fluorescent primer DNA | 500 nM | 2 µL |

The Activity buffer comprises, for example, TdT reaction buffer (available from New England Biolabs) supplemented with CoCl₂. Primer used is the following:
5'-AAAAAAAAAAAAAAGGGG-3' (SEQ ID NO:7)

The primer has also an ATTO fluorescent dye on the 5' extremity.

Nucleotides used (noted as dNTP in table 5) are 3'-O-amino-2',3'-dideoxynucleotides-5'-triphosphate (ONH₂, Firebird Biosciences) such as 3'-O-amino-2',3'-dideoxyadenosine-5'-triphosphate for example.

For each different variant tested, one tube is used for the reaction. The reagents are added in the tube, starting from water, and then in the order of Table 5. After 30 min at 37°C the reaction is stopped by addition of formamide (Sigma).

### Analysis

The analysis is involving polyacrylamide gel analysis. Samples from activity test are analyzed through polyacrylamide 16% (biorad) denaturing gel. Gels are made just before the analysis by pouring polyacrylamide inside glass plates and let it polymerize. The gel inside the glass plates is mounted on an adapted tank filed with TBE buffer (Sigma) for the electrophoresis step. The samples to be analyzed are loaded on the top of the gel. A tension of 500 to 2,000V is applied between the top and bottom of the gel for 3 to 6h at room temperature. Once migrated according to the sample target size, system is dismounted, and gel fluorescence is scanned through the use of Typhoon instrument (GE Life Sciences). After image acquisition, ImageJ software (imagej.nih.gov/ij/) is used to analyze the percentage of incorporation of the modified nucleotides.

### Example 3 - Evaluation of the activity of variants of TdT with unlabeled primer

### Activity Test

Elongation performance of variants 5 generated, expressed and purified according to example 1 is evaluated through the following assay. All the results are compared with a reference variant (SEQ ID NO:9) obtained from previous research and to a control tube lacking any polymerase enzyme.

**Table 6: Activity test**

| **Reagent** | **Concentration** | **Volume** |
|---|---|---|
| H₂O | - | 12 µL |
| Activity Buffer | 10x | 2 µL |
| dNTP | 250 µM | 2 µL |
| Purified enzyme | 20 µM | 2 µL |
| Fluorescent primer DNA | 500 nM | 2 µL |

Primer used is the following:
5'- TTTTTTTTTTTTAAATAAGG-3' (SEQ ID NO:8)

Nucleotides used (noted as dNTP in table 6) were 3'-O-amino-2',3'-dideoxynucleotides-5'-triphosphate (ONH2, Firebird Biosciences) such as 3'-O-amino-2',3'-dideoxyadenosine-5'-triphosphate for example.

For each variant tested one tube was used for the reaction. The reagents are added in the tube starting from the water and then in the order of Table 6. After 30min at 37°C the reaction was stopped by addition of formamide (Sigma).

### Analysis

The analysis used liquid chromatography and mass spectrometer detection and quantification (LC/MS). Samples from activity test are analyzed through LC/MS. Samples are loaded into the LC/MS instrument and a standard oligonucleotide separation method is performed. Acquisition of data was followed by deconvolution and spectrum calculation.

## Claims

1. A terminal deoxynucleotidyl transferase (TdT) variant comprising an amino acid sequence at least 90% identical to the amino acid sequence as set forth in the amino acid sequence selected from SEQ ID NO: 11, 13, 15, 17, 19, 21, 23, 25, 27, 29, 31, 33 or 35,
- wherein the amino acid sequence comprises at least one amino acid substitution of a cysteine residue at position 173 with respect to SEQ ID NOs 11, 13, 17, 19, 21, 29 or 31, or of a functionally equivalent cysteine residue selected from a cysteine at position 172 with respect to SEQ ID NO: 15, or cysteine at position 178 with respect to SEQ ID NO: 23, or cysteine at position 174 with respect to SEQ ID NO: 25, or cysteine at position 171 with respect to SEQ ID NO: 27, or cysteine at position 182 with respect to SEQ ID NO: 33, or cysteine at position 176 with respect to SEQ ID NO: 35,
- wherein the amino acid sequence comprises a further substitution of an arginine residue at position 207 with respect to SEQ ID NOs 11, 13, 17, 19, 23, 29 of 31, or of a functionally equivalent arginine residue selected from an arginine at position 206 with respect to SEQ ID NO: 15, or a arginine at position 208 with respect to SEQ ID NOs 21 or 25, or arginine at position 205 with respect to SEQ ID NO: 27, or arginine at position 216 with respect to SEQ ID NO: 33, or a arginine at position 210 with respect to SEQ ID NO: 35,
wherein the TdT variant (i) is capable of synthesizing a nucleic acid fragment without a template and (ii) is capable of incorporating a modified nucleotide into a nucleic acid fragment.

2. The TdT variant of claim 1 having a further substitution of a methionine residue at position 63 with respect to SEQ ID NOs 11, 13, 15, 17, 19, 29 or 33, or of a functionally equivalent methionine residue selected from a methionine at position 73 with respect to SEQ ID NO: 23, or methionine at position 73 with respect to SEQ ID NO: 23, or methionine at position 64 with respect to SEQ ID NO: 25, or methionine at position 61 with respecti to SEQ ID NO: 27, or a methionine at position 66 with respect to SEQ ID NO: 35.

3. The TdT variant of claim 1 or 2, having a further substitution of an arginine residue at position 324 with respect to SEQ ID NOs 11 or 13, or of a functionally equivalent arginine residue selected from an arginine at position 320 with respect to SEQ ID NO: 15, or a arginine at position 331 with respect to SEQ ID NO: 17, or a arginine at position 325 with respect to SEQ ID NOs 19, 23 or 31, or a arginine at position 326 with respect to SEQ ID NO: 25, or a arginine at position 323 with respect to SEQ ID NO: 27, or a arginine at position 328 with respect to SEQ ID NO: 29, or a arginine at position 338 with respect to SEQ ID NO: 33, or a arginine at position 328 with respect to SEQ ID NO: 35.

4. The TdT variant of any one of the previous claims, having a further substitution of glutamic acid residue at position 327 with respect to SEQ ID NOs 11or 13, or of a functionally equivalent glutamic acid residue selected from a glutamic acid at position 334 with respect to SEQ ID NO: 17 or 21, or a glutamic acid at position 331 with respect to SEQ ID NOs 29 or 35, or a glutamic acid at position 328 with respect to SEQ ID NOs 19, 23 or 31, or a glutamic acid at position 329 with respect to SEQ ID NO: 25, or a glutamic acid at position 326 with respect to SEQ ID NO: 27, or a glutamic acid at position 341 with respect to SEQ ID NO: 33.

5. The TdT variant of any one of the previous claims, wherein said substitution of said cysteine is G, R, P, A, V, S, N, Q or D, preferably G or R.

6. The TdT variant of any one of claim 2 to 5, wherein said substitution of said methionine is R, Q, G, A, V, D, N, H or E, preferably R or Q.

7. The TdT variant of any one of the previous claims, wherein said substitution of said arginine is N, L, K, H, G, D, A or P, preferably N or L.

8. The TdT variant of any one of claim 4 to 7, wherein said substitution of said glutamic acid is N, L, T or S.

9. The TdT variant of any one of claims 1 to 8, wherein said TdT variant is capable of incorporating onto a nucleic acid fragment, preferably onto a free 3'-hydroxyl of a nucleice acid fragment, a 3'-O-modified nucleotide, preferably selected from a 3'-O-NH₂-nucleoside triphosphate, a 3'-O-azidomethyl-nucleoside triphosphate, a 3'-O-allyl-nucleoside triphosphate, a 3'O-(2-nitrobenzyl)-nucleoside triphosphate, or a 3'-O-propargyl-nucleoside triphosphate, more preferably a 3'-O-NH₂-nucleoside triphosphate.

10. The TdT variant of any one of claims 1 to 9, wherein said TdT variant incorporates said 3'-O-modified nucleotide at a rate greater than that of a wild type TdT.

11. A nucleic acid encoding a TdT variant as defined in any one of claims 1 to 10.

12. A method of producing a TdT variant comprising:
(a) culturing a host cell comprising a nucleic acid according to claim 11 under conditions suitable to express the nucleic acid encoding the TdT variant; and optionally
(b) recovering said TdT variant from the cell culture.

13. A method of synthesizing a polynucleotide having a predetermined sequence, the method comprising the steps of:
a) providing an initiator having a 3'-terminal nucleotide having a free 3'-hydroxyl;
b) repeating cycles of (i) contacting under elongation conditions the initiator or elongated fragments having free 3'-O-hydroxyls with a 3'-O-blocked nucleoside triphosphate and a TdT variant according to any one of claims 1 to 10, so that the initiator or elongated fragments are elongated by incorporation of a 3'-O-blocked nucleoside triphosphate to form 3'-O-blocked elongated fragments, and (ii) deblocking the elongated fragments to form elongated fragments having free 3'-hydroxyls, until the polynucleotide is formed.

14. The method of claim 13 wherein said 3'-O-blocked nucleoside triphosphate is a 3'-O-NH₂-nucleoside triphosphate, a 3'-O-azidomethyl-nucleoside triphosphate, a 3'-O-allyl-nucleoside triphosphate, or a 3'-O-(2-nitrobenzyl)-nucleoside triphosphate.

15. A kit for performing a nucleotide incorporation reaction comprising
- a variant of TdT according to any one of claims 1 to 10,
- one or more nucleotides, preferably one or more 3'O-modified nucleotides, and
- optionally at least one nucleic acid primer.

## Patentansprüche

1. Terminale Desoxynukleotidyl-Transferase (TdT)-Variante, umfassend eine Aminosäuresequenz, die zu mindestens 90% identisch ist mit der Aminosäuresequenz, wie sie in der Aminosäuresequenz, ausgewählt aus SEQ ID NO: 11, 13, 15, 17, 19, 21, 23, 25, 27, 29, 31, 33 oder 35, angegeben ist,
- wobei die Aminosäuresequenz mindestens eine Aminosäuresubstitution eines Cystein-Restes an Position 173 in Bezug auf SEQ ID NOs 11, 13, 17, 19, 21, 29 oder 31 oder eines funktionell äquivalenten Cystein-Restes umfasst, der aus einem Cystein an Position 172 in Bezug auf SEQ ID NO: 15, oder Cystein an Position 178 in Bezug auf SEQ ID NO: 23, oder Cystein an Position 174 in Bezug auf SEQ ID NO: 25, oder Cystein an Position 171 in Bezug auf SEQ ID NO: 27, oder Cystein an Position 182 in Bezug auf SEQ ID NO: 33, oder Cystein an Position 176 in Bezug auf SEQ ID NO: 35 ausgewählt ist,
- wobei die Aminosäuresequenz eine weitere Substitution eines Arginin-Restes an Position 207 in Bezug auf SEQ ID NOs 11, 13, 17, 19, 23, 29 oder 31 oder eines funktionell äquivalenten Arginin-Restes umfasst, der aus einem Arginin an Position 206 in Bezug auf SEQ ID NO: 15, oder einem Arginin an Position 208 in Bezug auf SEQ ID NOs 21 oder 25, oder Arginin an Position 205 in Bezug auf SEQ ID NO: 27, oder Arginin an Position 216 in Bezug auf SEQ ID NO: 33, oder einem Arginin an Position 210 in Bezug auf SEQ ID NO: 35 ausgewählt ist,
wobei die TdT-Variante (i) in der Lage ist, ein Nukleinsäurefragment ohne eine Matrize zu synthetisieren, und (ii) in der Lage ist, ein modifiziertes Nukleotid in ein Nukleinsäurefragment einzubauen.

2. TdT-Variante nach Anspruch 1, die eine weitere Substitution eines Methionin-Restes an Position 63 in Bezug auf SEQ ID NOs 11, 13, 15, 17, 19, 29 oder 33 oder eines funktionell äquivalenten Methionin-Restes umfasst, der aus einem Methionin an Position 73 in Bezug auf SEQ ID NO: 23, oder Methionin an Position 73 in Bezug auf SEQ ID NO: 23, oder Methionin an Position 64 in Bezug auf SEQ ID NO: 25, oder Methionin an Position 61 in Bezug auf SEQ ID NO: 27, oder einem Methionin an Position 66 in Bezug auf SEQ ID NO: 35 ausgewählt ist.

3. TdT-Variante nach Anspruch 1 oder 2, die eine weitere Substitution eines Arginin-Restes an Position 324 in Bezug auf SEQ ID NO: 11 oder 13 oder eines funktionell äquivalenten Arginin-Restes umfasst, der aus einem Arginin an Position 320 in Bezug auf SEQ ID NO: 15, oder einem Arginin an Position 331 in Bezug auf SEQ ID NO: 17, oder einem Arginin an Position 325 in Bezug auf SEQ ID NOs: 19, 23 oder 31, oder einem Arginin an Position 326 in Bezug auf SEQ ID NO: 25, oder einem Arginin an Position 323 in Bezug auf SEQ ID NO: 27, oder einem Arginin an Position 328 in Bezug auf SEQ ID NO: 29, oder einem Arginin an Position 338 in Bezug auf SEQ ID NO: 33, oder einem Arginin an Position 328 in Bezug auf SEQ ID NO: 35 ausgewählt ist

4. TdT-Variante nach einem der vorhergehenden Ansprüche, die eine weitere Substitution eines Glutaminsäure-Restes an Position 327 in Bezug auf SEQ ID NOs 11 oder 13 oder eines funktionell äquivalenten Glutaminsäure-Restes umfasst, ausgewählt aus einer Glutaminsäure an Position 334 in Bezug auf SEQ ID NOs: 17 oder 21, oder einer Glutaminsäure an Position 331 in Bezug auf die SEQ ID NOs 29 oder 35, oder einer Glutaminsäure an Position 328 in Bezug auf die SEQ ID NOs 19, 23 oder 31, oder einer Glutaminsäure an Position 329 in Bezug auf die SEQ ID NO: 25, oder einer Glutaminsäure an Position 326 in Bezug auf die SEQ ID NO: 27, oder einer Glutaminsäure an Position 341 in Bezug auf die SEQ ID NO: 33 ausgewählt ist.

5. TdT-Variante nach einem der vorhergehenden Ansprüche, wobei die Substitution des Cysteins G, R, P, A, V, S, N, Q oder D ist, vorzugsweise G oder R.

6. TdT-Variante nach einem der Ansprüche 2 bis 5, wobei die Substitution des Methionins R, Q, G, A, V, D, N, H oder E ist, vorzugsweise R oder Q.

7. TdT-Variante nach einem der vorhergehenden Ansprüche, wobei die Substitution des Arginins N, L, K, H, G, D, A oder P ist, vorzugsweise N oder L.

8. TdT-Variante nach einem der Ansprüche 4 bis 7, wobei die Substitution der Glutaminsäure N, L, T oder S ist.

9. TdT-Variante nach einem der Ansprüche 1 bis 8, wobei die TdT-Variante in der Lage ist, an ein Nukleinsäurefragment, vorzugsweise an ein freies 3'-Hydroxyl eines Nukleinsäurefragments, ein 3'-O-modifiziertes Nukleotid einzubauen, vorzugsweise ausgewählt aus einem 3'-O-NH₂-Nukleosidtriphosphat, einem 3'-O-Azidomethyl-Nukleosidtriphosphat, einem 3'-O-Allyl-Nukleosidtriphosphat, einem 3'-O-(2-Nitrobenzyl)-Nukleosidtriphosphat oder einem 3'-O-Propargyl-Nukleosidtriphosphat, besonders bevorzugt einem 3'-O-NH₂-Nukleosidtriphosphat.

10. TdT-Variante nach einem der Ansprüche 1 bis 9, wobei die TdT-Variante das 3'-O-modifizierte Nukleotid mit einer höheren Rate als der einer Wildtyp-TdT einbaut.

11. Nukleinsäure, die für eine TdT-Variante nach einem der Ansprüche 1 bis 10 kodiert.

12. Verfahren zur Herstellung einer TdT-Variante, umfassend:
(a) Kultur einer Wirtszelle, die eine Nukleinsäure nach Anspruch 11 umfasst, unter Bedingungen, die geeignet sind, die Nukleinsäure, die für die TdT-Variante kodiert, zu exprimieren; und gegebenenfalls
(b) Gewinnen der TdT-Variante aus der Zellkultur.

13. Verfahren zur Synthese eines Polynukleotids mit einer vorbestimmten Sequenz, wobei das Verfahren die folgenden Schritte umfasst:
a) Bereitstellen eines Initiators mit einem 3'-terminalen Nukleotid, das ein freies 3'-Hydroxyl aufweist;
b) Wiederholen von Zyklen (i) In-Kontakt-Bringen des Initiators oder verlängerter Fragmente mit freien 3'-O-Hydroxylen mit einem 3'-O-blockierten Nukleosidtriphosphat und einer TdT-Variante nach einem der Ansprüche 1 bis 10 unter Verlängerungsbedingungen, so dass der Initiator oder die verlängerten Fragmente durch Einbau eines 3'-O-blockierten Nukleosidtriphosphats verlängert werden, um 3'-O-blockierte verlängerte Fragmente zu bilden, und (ii) Entblockieren der verlängerten Fragmente, um verlängerte Fragmente mit freien 3'-Hydroxylen zu bilden, bis das Polynukleotid gebildet ist.

14. Verfahren nach Anspruch 13, wobei das 3'-O-blockierte Nukleosidtriphosphat ein 3'-O-NH₂-Nukleosidtriphosphat, ein 3'-O-Azidomethyl-Nukleosidtriphosphat, ein 3'-O-Allyl-Nukleosidtriphosphat oder ein 3'-O-(2-Nitrobenzyl)-Nukleosidtriphosphat ist.

15. Kit zur Durchführung einer Nukleotid-Einbaureaktion, umfassend:
- eine TdT-Variante nach einem der Ansprüche 1 bis 10,
- ein oder mehrere Nukleotide, vorzugsweise ein oder mehrere 3'-O-modifizierte Nukleotide, und
- gegebenenfalls mindestens einen Nukleinsäureprimer.

## Revendications

1. Variant de désoxynucléotidyl transférase terminale (TdT) comprenant une séquence d'acides aminés identique à au moins 90 % à la séquence d'acides aminés telle que représentée par la séquence d'acides aminés sélectionnée parmi SEQ ID NO : 11, 13, 15, 17, 19, 21, 23, 25, 27, 29, 31, 33 ou 35,
- dans lequel la séquence d'acides aminés comprend au moins une substitution d'acide aminé d'un résidu cystéine en position 173 par rapport à SEQ ID NO 11, 13, 17, 19, 21, 29 ou 31, ou d'un résidu cystéine fonctionnellement équivalent sélectionné parmi une cystéine en position 172 par rapport à SEQ ID NO : 15, ou une cystéine en position 178 par rapport à SEQ ID NO : 23, ou une cystéine en position 174 par rapport à SEQ ID NO : 25, ou une cystéine en position 171 par rapport à SEQ ID NO : 27, ou une cystéine en position 182 par rapport à SEQ ID NO : 33, ou une cystéine en position 176 par rapport à SEQ ID NO : 35,
- dans lequel la séquence d'acides aminés comprend une substitution supplémentaire d'un résidu arginine en position 207 par rapport à SEQ ID NO 11, 13, 17, 19, 23, 29 ou 31, ou d'un résidu arginine fonctionnellement équivalent sélectionné parmi une arginine en position 206 par rapport à SEQ ID NO : 15, ou une arginine en position 208 par rapport à SEQ ID NO 21 ou 25, ou une arginine en position 205 par rapport à SEQ ID NO : 27, ou une arginine en position 216 par rapport à SEQ ID NO : 33, ou une arginine en position 210 par rapport à SEQ ID NO : 35,
dans lequel le variant de TdT (i) est capable de synthétiser un fragment d'acide nucléique sans matrice et (ii) est capable d'incorporer un nucléotide modifié dans un fragment d'acide nucléique.

2. Variant de TdT selon la revendication 1, ayant une substitution supplémentaire d'un résidu méthionine en position 63 par rapport à SEQ ID NO 11, 13, 15, 17, 19, 29 ou 33, ou d'un résidu méthionine fonctionnellement équivalent sélectionné parmi une méthionine en position 73 par rapport à SEQ ID NO : 23, ou une méthionine en position 73 par rapport à SEQ ID NO : 23, ou une méthionine en position 64 par rapport à SEQ ID NO : 25, ou une méthionine en position 61 par rapport à SEQ ID NO : 27, ou une méthionine en position 66 par rapport à SEQ ID NO : 35.

3. Variant de TdT selon la revendication 1 ou 2, ayant une substitution supplémentaire d'un résidu arginine en position 324 par rapport à SEQ ID NO 11 ou 13, ou d'un résidu arginine fonctionnellement équivalent sélectionné parmi une arginine en position 320 par rapport à SEQ ID NO : 15, ou une arginine en position 331 par rapport à SEQ ID NO : 17, ou une arginine en position 325 par rapport à SEQ ID NO 19, 23 ou 31, ou une arginine en position 326 par rapport à SEQ ID NO : 25, ou une arginine en position 323 par rapport à SEQ ID NO : 27, ou une arginine en position 328 par rapport à SEQ ID NO : 29, ou une arginine en position 338 par rapport à SEQ ID NO : 33, ou une arginine en position 328 par rapport à SEQ ID NO : 35.

4. Variant de TdT selon l'une quelconque des revendications précédentes, ayant une substitution supplémentaire d'un résidu acide glutamique en position 327 par rapport à SEQ ID NO 11 ou 13, ou d'un résidu acide glutamique fonctionnellement équivalent sélectionné parmi un acide glutamique en position 334 par rapport à SEQ ID NO : 17 ou 21, ou un acide glutamique en position 331 par rapport à SEQ ID NO 29 ou 35, ou un acide glutamique en position 328 par rapport à SEQ ID NO 19, 23 ou 31, ou un acide glutamique en position 329 par rapport à SEQ ID NO : 25, ou un acide glutamique en position 326 par rapport à SEQ ID NO : 27, ou un acide glutamique en position 341 par rapport à SEQ ID NO : 33.

5. Variant de TdT selon l'une quelconque des revendications précédentes, dans lequel ladite substitution de ladite cystéine est G, R, P, A, V, S, N, Q ou D, de préférence G ou R.

6. Variant de TdT selon l'une quelconque des revendications 2 à 5, dans lequel ladite substitution de ladite méthionine est R, Q, G, A, V, D, N, H ou E, de préférence R ou Q.

7. Variant de TdT selon l'une quelconque des revendications précédentes, dans lequel ladite substitution de ladite arginine est N, L, K, H, G, D, A ou P, de préférence N ou L.

8. Variant de TdT selon l'une quelconque des revendications 4 à 7, dans lequel ladite substitution dudit acide glutamique est N, L, T ou S.

9. Variant de TdT selon l'une quelconque des revendications 1 à 8, dans lequel ledit variant de TdT est capable d'incorporer sur un fragment d'acide nucléique, de préférence sur un 3'-hydroxyle libre d'un fragment d'acide nucléique, un nucléotide 3'-O-modifié, de préférence sélectionné parmi un 3'-O-NH₂-nucléoside triphosphate, un 3'-O-azidométhyl-nucléoside triphosphate, un 3'-O-allyl-nucléoside triphosphate, un 3'-O-(2-nitrobenzyl)-nucléoside triphosphate, ou un 3'-O-propargyl-nucléoside triphosphate, de manière davantage préférée un 3'-O-NH₂-nucléoside triphosphate.

10. Variant de TdT selon l'une quelconque des revendications 1 à 9, dans lequel ledit variant de TdT incorpore ledit nucléotide 3'-O-modifié à un taux supérieur à celui d'une TdT de type sauvage.

11. Acide nucléique codant pour un variant de TdT tel que défini dans l'une quelconque des revendications 1 à 10.

12. Procédé de production d'un variant de TdT comprenant :
(a) la culture d'une cellule hôte comprenant un acide nucléique selon la revendication 11 dans des conditions appropriées pour exprimer l'acide nucléique codant pour le variant de TdT ; et facultativement
(b) la récupération dudit variant de TdT à partir de la culture cellulaire.

13. Procédé de synthèse d'un polynucléotide ayant une séquence prédéterminée, le procédé comprenant les étapes suivantes :
a) la fourniture d'un initiateur ayant un nucléotide 3'-terminal ayant un 3'-hydroxyle libre ;
b) la répétition des cycles de (i) mise en contact, dans des conditions d'élongation, de l'initiateur ou de fragments allongés ayant des 3'-O-hydroxyles libres avec un nucléoside triphosphate 3'-O-bloqué et d'un variant de TdT selon l'une quelconque des revendications 1 à 10, de sorte l'initiateur ou les fragments allongés sont allongés par l'incorporation d'un nucléoside triphosphate 3'-O-bloqué pour former des fragments allongés 3'-O-bloqués, et (ii) déblocage des fragments allongés pour former des fragments allongés ayant des 3'-hydroxyles libres, jusqu'à ce que le polynucléotide soit formé.

14. Procédé selon la revendication 13, dans lequel ledit nucléoside triphosphate 3'-O-bloqué est un 3'-O-NH₂-nucléoside triphosphate, un 3'-O-azidométhyl-nucléoside triphosphate, un 3'-O-allyl-nucléoside triphosphate, ou un 3'-O-(2-nitrobenzyl)-nucléoside triphosphate.

15. Kit pour mettre en oeuvre une réaction d'incorporation de nucléotide comprenant
- un variant de TdT selon l'une quelconque des revendications 1 à 10,
- un ou plusieurs nucléotides, de préférence un ou plusieurs nucléotides 3'-O-modifiés, et
- facultativement au moins une amorce d'acide nucléique.
